# EUROPÄISCHE PATENTSCHRIFT

(11) **EP 4 000 656 B1**
(45) Veröffentlichungstag und Bekanntmachung des Hinweises auf die Patenterteilung: **21.08.2024**
(21) Anmeldenummer: 21206366.3
(22) Anmeldetag: 04.11.2021
(51) Int. Cl.: B01D 47/02, B01D 53/00, B01D 53/72, A61L 9/20, B01D 46/00, B01D 46/10, B01D 50/60

(54) **VORRICHTUNG UND VERFAHREN ZUR REDUZIERUNG EINER SCHADSTOFFBELASTUNG IN EINEM GAS**
DEVICE AND METHOD FOR REDUCING THE AMOUNT OF POLLUTANTS IN A GAS
DISPOSITIF ET PROCÉDÉ DE RÉDUCTION DE LA CHARGE EN SUBSTANCES NOCIVES DANS UN GAZ

(30) Priorität: 13.11.2020 DE 102020130003
(43) Veröffentlichungstag der Anmeldung: 25.05.2022
(73) Patentinhaber: Richter lighting technologies GmbH, 73540 Heubach (DE)
(72) Erfinder: Richter, Bernd, 73540 Heubach (DE); Röhrer, Sebastian, 73431 Aalen (DE); Tischer, Dr. Ingo, 73563 Mögglingen (DE)
(74) Vertreter: Lorenz, Matthias

(56) Entgegenhaltungen:
- WO-A1-2007/064188
- WO-A1-2007/138948
- WO-A1-2015/059336
- WO-A1-2017/218832
- DE-A1- 3 630 710
- RU-C1- 2 594 088
- US-A1- 2004 005 252

## Beschreibung

Die Erfindung betrifft eine Vorrichtung zur Reduzierung einer Schadstoffbelastung in einem Gas, aufweisend einen Gaseinlass und einen Gasauslass, sowie einen dazwischen angeordneten Desinfektionsraum, gemäß dem Oberbegriff des Anspruchs 1.

Außerdem betrifft die Erfindung ein System zur Beeinflussung eines Gasstroms, aufweisend eine Basiseinheit mit wenigstens einer Strömungsmaschine und einer Vorrichtung zur Reduzierung einer Schadstoffbelastung in dem Gas, die als Erweiterung der Basiseinheit mit der Basiseinheit verbunden ist.

Die Erfindung betrifft ferner ein Verfahren zur Reduzierung einer Schadstoffbelastung in einem Gas, gemäß dem Oberbegriff des Anspruchs 13.

Bekanntermaßen weist Raumluft in geschlossenen oder wenig belüfteten Räumen in der Regel erheblich mehr Schadstoffe auf als Außenluft. Vorrichtungen zur Reduzierung einer solchen Schadstoffbelastung sind unter anderem für den Haushaltsgebrauch und für gewerbliche Anwendungen bekannt. Die bekannten Luftreiniger vermögen die Raumluft beispielsweise von Krankheitserregern, Staub und Allergenen zu befreien oder deren Vorkommen in der Raumluft zumindest bis zu einem verträglichen bzw. unschädlichen Maß zu reduzieren.

Die bekannten Luftreiniger setzen in der Regel eine Kombination verschiedener mechanischer Filter (beispielsweise Schwebstofffilter) und Aktivkohlefilter sowie optional eine zusätzliche UV-C-Bestrahlung ein.

Um die Filterwirkung zu verbessern und zusätzlich den Luftdurchsatz zu erhöhen sind außerdem Vorrichtungen zur Luftreinigung bekannt, bei denen die Reinigung durch die Emission von Hydroxylradikalen erfolgt. Eine solche Vorrichtung ist beispielsweise in der WO 2015/059336 A1 beschrieben. Die Vorrichtung der WO 2015/059336 A1 weist eine Kartusche mit einem flüssigen Reaktanten auf, der durch Reaktion mit ultravioletter Strahlung Hydroxylradikale erzeugen kann. Ausgehend von der Kartusche erstreckt sich ein Docht, der in den Luftstrom hineinragt. Der Docht wird somit von dem Luftstrom umspült, wodurch der Reaktant teilweise an die Luft abgegeben wird. Anschließend werden mittels eines UV-Strahlgenerators in dem Aerosol Hydroxylradikale erzeugt. Die Hydroxylradikale vermögen in bekannter Weise organische Verunreinigungen und damit in erster Linie die in der Luft enthaltenen Schadstoffe abzubauen. Diese Art der Desinfektion erfolgt schnell und hochwirksam.

Ein Problem der beschriebenen Vorrichtung ist allerdings die in der Praxis nur geringe Freisetzung des Reaktanten über den Docht. Die Reinigungswirkung ist aus diesem Grunde nicht zufriedenstellend.

Um die Aerosolbildung mit dem flüssigen Reaktanten zu verbessern sind im Stand der Technik auch Alternativen zu der Abgabe des Reaktanten an die Luft bekannt. Beispielsweise wird in der CN 208809155 U vorgeschlagen, den Luftstrom durch den flüssigen Reaktanten hindurch zu leiten. Als weitere Alternative wird in der US 2007/0217944 A1 vorgeschlagen, den Reaktanten zu versprühen.

Die genannten Maßnahmen sind technisch allerdings vergleichsweise aufwendig zu realisieren.

Es besteht somit ein Bedarf, die bekannten Luftreiniger weiter zu verbessern.

Zum weiteren technischen Hintergrund sei noch auf die folgenden Druckschriften verwiesen. Die WO 2007/138948 A1 betrifft eine Luftdesinfektionsvorrichtung unter Verwendung von elektrolysiertem Wasser in Kombination mit einem kapillaren Material. Die WO 2007/064188 A1 betrifft ein Duftabgabesystem mit einem mit einer Duftsubstanz gefüllten Duftreservoir. Die DE 36 30 710 A1 betrifft einen Absorptionsschalldämpfer, welcher in der Staubförderleitung eines Handstaubsaugers mit sogenanntem nachgeschaltetem Filter angeordnet ist, wobei die Staubförderleitung auf einem Teil ihres Umfangs durchbrochen ist, und dieses durchbrochene Teil von einem weichen Schallabsorptionsmaterial umgeben ist. Die RU 2 594 088 C1 betrifft eine Vorrichtung zur Schalldämpfung. Ferner betrifft die US 2004/0005252 A1 eine Vorrichtung zur Luftreinigung mittels eines durchströmbaren Nassfilters, um ein Oxidationsmittel abzusondern und den Luftstrom in Folge von schädlichen organischen Partikeln zu säubern.

In Anbetracht des bekannten Stands der Technik besteht die Aufgabe der vorliegenden Erfindung darin, eine Vorrichtung bereitzustellen, mittels der eine Schadstoffbelastung in einem Gas zuverlässig und bei vorzugsweise hohem Gasdurchsatz reduziert werden kann, insbesondere mit technisch einfachen Mitteln.

Der Erfindung liegt außerdem die Aufgabe zugrunde, ein System zur Beeinflussung eines Gasstroms bereitzustellen, bei dem eine Basiseinheit, die eine Strömungsmaschine aufweist, mit einer vorteilhaften Vorrichtung zur Reduzierung einer Schadstoffbelastung in einem Gas erweitert werden kann.

Der vorliegenden Erfindung liegt auch die Aufgabe zugrunde, ein Verfahren bereitzustellen, mit dem eine Schadstoffbelastung in einem Gas zuverlässig und bei vorzugsweise hohem Gasdurchsatz reduziert werden kann, insbesondere mit technisch einfachen Mitteln.

Die Aufgabe wird für die Vorrichtung mit den in Anspruch 1 aufgeführten Merkmalen gelöst. Betreffend das System wird die Aufgabe durch Anspruch 12 gelöst. Hinsichtlich des Verfahrens wird die Aufgabe durch die Merkmale des Anspruchs 13 gelöst.

Die abhängigen Ansprüche und die nachfolgend beschriebenen Merkmale betreffen vorteilhafte Ausführungsformen und Varianten der Erfindung.

Es ist eine Vorrichtung zur Reduzierung einer Schadstoffbelastung in einem Gas (oder Gasgemisch) vorgesehen, aufweisend einen Gaseinlass und einen Gasauslass, sowie einen dazwischen angeordneten Desinfektionsraum, der von einem von dem Gaseinlass zu dem Gasauslass verlaufenden Gasstrom durchströmbar ist.

Bei Schadstoffen kann es sich im Rahmen der Erfindung um Krankheitserreger wie Viren, Bakterien oder Sporen, um Pathogene oder um sonstige Schadstoffe wie beispielsweise Pollen, Gerüche, Staub und Mikropartikel, flüchtige organische Verbindungen und/oder Schuppen handeln. Ganz besonders eignet sich die erfindungsgemäße Vorrichtung zur Reduzierung einer organischen Schadstoffbelastung.

Bei dem von den Schadstoffen zu befreienden Gas bzw. Gasgemisch kann es sich insbesondere um Luft, vorzugsweise um Raumluft handeln.

An dieser Stelle sei erwähnt, dass im Rahmen der Erfindung auch mehrere Gaseinlässe und/oder mehrere Gasauslässe vorgesehen sein können.

Vorzugsweise erfolgt die Reduzierung der Schadstoffbelastung im Wesentlichen innerhalb des Desinfektionsraums, besonders bevorzugt ausschließlich innerhalb des Desinfektionsraums. Erfindungsgemäß ist vorgesehen, dass eine ein kapillares Material aufweisende Aerosolerzeugungsanordnung innerhalb des Desinfektionsraums von dem Gasstrom durchströmbar angeordnet ist.

Unter einer Aerosolerzeugungsanordnung ist insbesondere eine Anordnung zu verstehen, die eine Vermischung des Oxidationsmittels mit dem von den Schadstoffen zu befreienden Gas bewirkt. Vorzugsweise erzeugt die Aerosolerzeugungsanordnung ein Aerosol aus dem Oxidationsmittel und dem Gas. Grundsätzlich kann die Aerosolerzeugungsanordnung allerdings ein beliebiges Gemisch aus dem Gas und flüssigen, festen und/oder gasförmigen Teilchen des Oxidationsmittels erzeugen. Beispielsweise kann auch ein Dampf erzeugt werden.

Unter einem kapillaren Material ist insbesondere ein Material zu verstehen, das bei Kontakt mit einer Flüssigkeit einen Kapillareffekt auf dieselbe bewirkt, wodurch die Flüssigkeit in dem kapillaren Material ohne äußere Einflussnahme aufzusteigen vermag bzw. wodurch sich das kapillare Material mit der Flüssigkeit vollzusaugen vermag.

Vorzugsweise ist das kapillare Material in der Aerosolerzeugungsanordnung derart verteilt, dass es dem vorbeiströmenden Gasstrom insgesamt eine möglichst große Oberfläche zur Verfügung stellt.

Vorzugsweise ist das kapillare Material in der Aerosolerzeugungsanordnung verteilt angeordnet.

Insbesondere kann vorgesehen sein, dass die Aerosolerzeugungsanordnung von dem Gasstrom durchströmbar ist, jedoch nicht das kapillare Material selbst. Das kapillare Material soll vorzugsweise nicht als Nassfilter fungieren. Vorzugsweise ist das kapillare Material derart angeordnet, dass der Gasstrom parallel bzw. entlang dessen Oberfläche vorbeiströmen kann, nicht jedoch die Oberfläche durchströmt. Auf diese Weise kann ein besonders hoher Volumenstrom möglich sein.

Optional kann ein sich entlang seiner Längsachse zumindest abschnittsweise in den Desinfektionsraum erstreckender Gaseinleitungsstutzen vorgesehen sein. Der Gaseinleitungsstutzen kann ausgebildet sein, um das Gas ausgehend von dem Gaseinlass in den Desinfektionsraum zu leiten. Zwischen dem Gaseinlass und dem Gaseinleitungsstutzen kann vorzugsweise die nachfolgend noch beschriebene Strömungsmaschine angeordnet sein.

Erfindungsgemäß ist weiter vorgesehen, dass sich das kapillare Material in ein Flüssigkeitsreservoir erstreckt, in dem ein flüssiges Oxidationsmittel enthalten ist.

Das flüssige Oxidationsmittel kann in seiner reinen Form oder als Gemisch mit einer weiteren Flüssigkeit, insbesondere als Gemisch mit einem weiteren Oxidationsmittel, Wasser, Alkohol und/oder einem zusätzlichen Desinfektionsmittel, vorliegen.

Dadurch, dass sich das kapillare Material in das Flüssigkeitsreservoir erstreckt, steht es mit dem flüssigen Oxidationsmittel in direkter Verbindung, vorzugsweise statisch bzw. unbeweglich, wodurch sich das kapillare Material aufgrund des Kapillareffekts selbstständig mit dem Oxidationsmittel vollsaugt, gegebenenfalls auch entgegen der Schwerkraft. Auf diese Weise kann das flüssige Oxidationsmittel innerhalb des Desinfektionsraums großflächig verteilt werden, ohne dass hierfür besondere technische Mittel (wie Aktoren, Pumpen, Düsen etc.) erforderlich wären.

Überraschend hat sich gezeigt, dass das an dem mit dem Oxidationsmittel benetzten kapillaren Material vorbeiströmende Gas eine starke Aerosolbildung des flüssigen Oxidationsmittels verursacht. Auf diese Weise kann das Oxidationsmittel mit technisch einfachen Mitteln umfassend innerhalb des Gases verteilt werden, in dem Gas desinfizierend wirken und damit dessen Schadstoffbelastung reduzieren. Es hat sich gezeigt, dass diese Reaktion äußerst schnell und zuverlässig erfolgt, weshalb mittels der erfindungsgemäßen Vorrichtung auch ein besonders hoher Gasdurchsatz erreicht werden kann.

Vorzugsweise ist das kapillare Material statisch bzw. unbeweglich innerhalb des Desinfektionsraums angeordnet / befestigt.

In einer weiterführenden Ausgestaltung der Erfindung kann auch vorgesehen sein, dass das kapillare Material durch ein Schlauch- oder Leitungssystem mit dem flüssigen Oxidationsmittel benetzt oder getränkt wird. Der Transport des flüssigen Oxidationsmittels kann entweder passiv erfolgen (insbesondere durch die Schwerkraft) oder durch eine geregelte oder ungeregelte Pumpeinheit. In diesem Fall kann das Reservoir vorteilhaft als Auffangbehälter für abtropfende Flüssigkeit dienen, die anschließend wieder durch die Kapillarwirkung des kapillaren Materials aufgenommen wird.

Gemäß einer ersten Variante der Erfindung ist vorgesehen, dass wenigstens eine Innenwandung des Desinfektionsraums zumindest teilweise mit dem kapillaren Material der Aerosolerzeugungsanordnung ausgekleidet ist. Vorzugsweise sind alle Innenwandungen des Desinfektionsraums zumindest teilweise mit dem kapillaren Material ausgekleidet.

Etwaige Ausnehmungen in den Innenwandungen zum Gaseinlass und zum Gasauslass sind vorzugsweise ausgespart und damit nicht von dem kapillaren Material bedeckt.

Das kapillare Material ist vorzugsweise stoffschlüssig mit der wenigstens einen Innenwandung verbunden, vorzugsweise verklebt. Das kapillare Material kann allerdings auch kraft- oder formschlüssig mit der wenigstens einen Innenwandung des Desinfektionsraums verbunden sein.

Es kann vorgesehen sein, dass das kapillare Material die wenigstens eine Innenwandung des Desinfektionsraums nicht vollständig abdeckt und sich beispielsweise nur über einen Teilbereich der Längserstreckung des Desinfektionsraums erstreckt.

Grundsätzlich kann das kapillare Material beliebig innerhalb der Aerosolerzeugungsanordnung verteilt angeordnet sein, vorzugsweise mit dem Ziel, dem vorbeiströmenden Gasstrom eine möglichst große Oberfläche zu präsentieren.

Erfindungsgemäß ist das kapillare Material innerhalb der Aerosolerzeugungsanordnung derart angeordnet, dass der Gasstrom zumindest im Wesentlichen parallel an der mit dem flüssigen Oxidationsmittel benetzten Oberfläche des kapillaren Materials vorbeizuströmen vermag.

Es kann auch vorgesehen sein, alternativ oder zusätzlich zu einem Auskleiden der wenigstens einen Innenwandung des Desinfektionsraums wenigstens eine Außenwandung eines sich in den Desinfektionsraum erstreckenden Gaseinleitungsstutzens zumindest teilweise mit dem kapillaren Material auszukleiden.

Es können Mittel zur Verwirbelung des Gases in dem Desinfektionsraum vorgesehen sein, insbesondere im Bereich des Gaseinlasses, beispielsweise auch im Bereich des Gaseinleitungsstutzens. Beispielsweise kann ein Luftleitblech oder können mehrere Luftleitbleche und/oder Luftleitlamellen vorgesehen sein. Die Verweilzeit und Expositionsdauer im Inneren des Desinfektionsraums kann auf diese Weise weiter verbessert sein.

Beispielsweise kann das kapillare Material alternativ oder zusätzlich zu der Auskleidung der wenigstens einen Innenwandung zwischen den Innenwandungen des Desinfektionsraums verteilt angeordnet sein, beispielsweise plattenförmig, zickzackförmig, spiralförmig, in der Art eines Hohlkörpers (beispielsweise ein konzentrisch offener Zylinder oder ein konzentrisch offener Quader) oder auf sonstige Weise. Auf diese Weise kann die Oberfläche zur Verteilung des Oxidationsmittels an das Gas weiter vergrößert werden.

Gemäß einer zweiten Variante der Erfindung, die zusätzlich oder alternativ zu der ersten Variante der Erfindung vorgesehen sein kann, weist die Aerosolerzeugungsanordnung wenigstens einen aus dem kapillaren Material gebildeten länglichen Körper (ein vorzugsweise schmaler Körper mit größerer Länge als Breite/Dicke) auf, der sich durch den Desinfektionsraum erstreckt.

Hierdurch kann die exponierte Oberfläche weiter vergrößert sein.

Bei dem länglichen Körper kann es sich beispielsweise um einen zylinderartigen Körper (Voll- oder Hohlzylinder), um Streifen oder Platten des kapillaren Materials und/oder um Fäden oder Geflechte aus dem kapillaren Material handeln. Vorzugsweise ist die Längsachse des länglichen Körpers parallel zu der Längsachse des Desinfektionsraums angeordnet.

Beispielsweise kann auch vorgesehen sein, den länglichen Körper ringförmig oder spiralförmig in dem Desinfektionsraum anzuordnen.

Vorzugsweise sind mehrere längliche Körper vorgesehen, die innerhalb des Desinfektionsraums verteilt angeordnet sind.

Die länglichen Körper können jeweils ein erstes (unteres) Ende und ein zweites (oberes) Ende aufweisen. Vorzugsweise ragen die länglichen Körper mit ihrem ersten Ende in das Flüssigkeitsreservoir und erstrecken sich ausgehend von dem ersten Ende bis zu dem zweiten Ende durch den Desinfektionsraum. Die länglichen Körper können beispielsweise im Bereich des zweiten Endes in dem Desinfektionsraum befestigt sein, insbesondere über eine gemeinsame Befestigungsplatte.

Gemäß einer Weiterbildung der Erfindung kann vorgesehen sein, dass das kapillare Material ein poröses, anorganisches Material ist.

Vorzugsweise ist das kapillare Material ein textiles Glasfasergebilde.

Grundsätzlich kann allerdings ein beliebiges kapillares Material vorgesehen sein, selbst ein organisches kapillares Material (z. B. Baumwolle). Ein organisches Material ist allerdings nicht bevorzugt, da ein anorganisches Material unter dem Einfluss des Oxidationsmittels in der Regel eine längere bis unbegrenzte Haltbarkeit aufweisen kann.

Es kann vorgesehen sein, dass das kapillare Material eine Keramik, eine Textilie, insbesondere ein Gewebe, Gestrick, Gewirke oder Gelege, ein Vlies oder Papier ist. Auch eine Kombination aus mehreren Materialien kann gegebenenfalls vorgesehen sein.

Besonders bevorzugt ist das kapillare Material ein Glasfasergeflecht oder eine Glasfasermatte.

Erfindungsgemäß ist vorgesehen, dass wenigstens eine Strahlungsquelle zur Erzeugung einer ionisierenden Strahlung innerhalb des Desinfektionsraums angeordnet ist. Vorzugsweise ist genau eine Strahlungsquelle vorgesehen, die insbesondere möglichst zentral innerhalb des Desinfektionsraums angeordnet ist.

Unter einer ionisierenden Strahlung kann im Rahmen der Erfindung insbesondere eine Strahlung mit einem Wellenlängenbereich von 400 nm und weniger, vorzugsweise 250 nm und weniger, zu verstehen sein.

Vorzugsweise ist eine UV-C-Strahlungsquelle zur Erzeugung einer UV-C-Strahlung vorgesehen.

Die Wellenlänge der ultravioletten Strahlung kann beispielsweise 100 nm bis 300 nm betragen. Auch Strahlung mit hiervon abweichenden Wellenlängen kann allerdings vorgesehen sein, insbesondere noch kurzwelligere Strahlung.

Vorzugsweise ist UV-C-Strahlung mit einer Wellenlänge von 240 nm bis 260 nm vorgesehen, insbesondere 254 nm bis 260 nm, besonders bevorzugt 258 nm, da dieser Wellenlängenbereich einen besonders stark keimtötenden Effekt aufweist. Die desinfizierende Wirkung ionisierender Strahlung, insbesondere von UV-C-Strahlung, ist bekannt, weshalb vorliegend auf weitere Ausführungen verzichtet wird.

Eine Kombination aus dem mittels des kapillaren Materials als Aerosol verteilen Oxidationsmittels und einer ionisierenden Strahlung kann die desinfizierende Wirkung der Vorrichtung beachtlich verstärken, insbesondere durch eine Radikalbildung aus dem flüssigen Oxidationsmittel durch die ionisierende Strahlung. Für diese Kombination hat sich als besonders geeignete Strahlung Ultraviolettstrahlung, ganz besonders kurzwellige Ultraviolettstrahlung, insbesondere UV-C-Strahlung (100 nm bis 280 nm), herausgestellt.

Vorzugsweise wird eine ionisierende Strahlung in einem Wellenlängenbereich verwendet, in dem kein oder nur in zu vernachlässigendem Maße Ozon erzeugt wird.

Die UV-C-Strahlungsquelle kann beispielsweise eine Quecksilberdampflampe, eine Quarzlampe, eine UV-Leuchtdiode oder einen Diodenlaser, eine UV-Kaltkathodenröhre oder eine sonstige künstliche UV-C-Quelle aufweisen. Insofern UV-C-Leuchtdioden vorgesehen sind, können diese vorzugsweise in einer Reihenanordnung, insbesondere in einer Bandanordnung, verteilt sein, um eine UV-C-Bestrahlung über eine möglichst große Längserstreckung bereitzustellen.

Die UV-C-Strahlungsquelle kann eine einstellbare Intensität und/oder Wellenlänge aufweisen und damit bedarfsweise anpassbar sein. Die Intensität und/oder Wellenlänge kann beispielsweise von einem Benutzer der Vorrichtung manuell und/oder von einer Steuereinrichtung der Vorrichtung automatisch anpassbar sein. Beispielsweise kann die Anpassung der Intensität und/oder Wellenlänge auf verschiedenen Reinigungsprogrammen für verschiedene Schadstoffarten und/oder verschiedene Leistungsstufen basieren, die diskret oder kontinuierlich einstellbar sein können (manuell oder automatisch).

Für verschiedene Schadstoffbelastungsarten (beispielsweise Viren, Bakterien, Sporen etc.) kann die optimale Wellenlänge der ionisierenden Strahlung, insbesondere der UV-C-Strahlung, sowie die tödliche Intensität variieren. Entsprechende Wellenlängen und Intensitäten zur Photolyse diverser Schadstoffe sind im Stand der Technik ausreichend bekannt, weshalb der Fachmann oder der Benutzer der Vorrichtung die UV-C-Strahlungsquelle bedarfsweise anpassen kann. Zu berücksichtigen sein kann auch der Abstand der Strahlungsquelle von den Schadstoffen.

Grundsätzlich gilt, je höher die Strahlungsleistung und je länger die Bestrahlungsdauer ist, desto größer ist die erreichte desinfizierende Wirkung.

Vorzugsweise ist die Vorrichtung ausgebildet, um ein Austreten der ionisierenden Strahlung, beispielsweise der UV-C-Strahlung, zu vermeiden. Vor allem UV-C-Strahlung kann mit technisch einfachen Mitteln abgeschirmt werden, da Festkörper von UV-C-Strahlung in der Regel nicht durchdrungen werden. Beispielsweise kann vorgesehen sein, den Gaseinlass und/oder den Gasauslass derart anzuordnen, dass eine direkte Sichtverbindung zu der Strahlungsquelle unterbrochen ist. Alternativ oder ergänzend können Abschirmbleche vorgesehen sein, beispielsweise ein Edelstahlblech oder ein sonstiges Blech.

In einer Weiterbildung der Erfindung kann vorgesehen sein, dass die Strahlungsquelle derart ausgebildet und/oder angeordnet ist, dass sich die erzeugte ionisierende Strahlung über den größten Teil (50% oder mehr, vorzugsweise 60% oder mehr, besonders bevorzugt 70% oder mehr, ganz besonders bevorzugt 80% oder mehr, noch weiter bevorzugt 90% oder mehr) des Desinfektionsraums ausbreitet.

Insbesondere kann vorgesehen sein, dass die Strahlungsquelle derart ausgebildet und/oder angeordnet ist, dass sich die erzeugte ionisierende Strahlung vollständig in dem Desinfektionsraum ausbreitet.

Auf diese Weise kann der vorhandene Desinfektionsraum für die Desinfektion durch die ionisierende Strahlung besonders gut ausgenutzt und ein hoher Gasdurchsatz ermöglicht werden.

In einer vorteilhaften Weiterbildung der Erfindung kann vorgesehen sein, dass die Strahlungsquelle zumindest abschnittsweise innerhalb der Aerosolerzeugungsanordnung angeordnet ist.

Vorzugsweise erstreckt sich die Strahlungsquelle innerhalb des Desinfektionsraums über denselben Längsabschnitt des Desinfektionsraums wie die Aerosolerzeugungsanordnung bzw. deren kapillares Material. Es kann aber gegebenenfalls auch ausreichend sein, wenn sich die Längserstreckung des kapillaren Materials lediglich abschnittsweise mit der Längserstreckung der Strahlungsquelle innerhalb des Desinfektionsraums überlappt.

Die Strahlungsquelle kann vornehmlich länglich ausgebildet sein. Die Strahlungsquelle kann längs oder quer zur Strömungsrichtung des Gasstroms ausgerichtet sein.

Grundsätzlich kann sogar vorgesehen sein, dass die Strahlungsquelle der Aerosolerzeugungsanordnung bzw. dem kapillaren Material in Strömungsrichtung lediglich nachgeordnet ist.

In einer Weiterbildung der Erfindung kann vorgesehen sein, dass die Längserstreckung des Desinfektionsraums zwischen dem Gaseinlass und dem Gasauslass größer ist als der Durchmesser des Desinfektionsraums. An dieser Stelle sei betont, dass der Desinfektionsraum einen beliebigen Querschnitt bzw. eine beliebige Grundfläche aufweisen kann. Aus der Formulierung "Durchmesser" des Desinfektionsraums ist nicht zwingend zu schlussfolgern, dass die Grundfläche des Desinfektionsraums rund sein muss; der Begriff "Durchmesser" ist daher auf einen nicht runden Querschnitt analog anzuwenden.

Insbesondere ein langer Desinfektionsraum hat sich als besonders geeignet herausgestellt, um die Schadstoffbelastung in dem Gas bei gleichzeitig hohem Volumenstrom zu reduzieren. Die Verweildauer des Gases innerhalb des Desinfektionsraums kann erhöht werden, wenn die Ausdehnung des Desinfektionsraums in Längsrichtung vergrößert wird.

In einer vorteilhaften Weiterbildung der Erfindung kann vorgesehen sein, dass der Desinfektionsraum zwischen dem Gaseinlass und dem Gasauslass einen im Wesentlichen linear verlaufenden Kanal oder einen gewundenen Kanal für den Gasstrom ausbildet.

Ein linearer Kanal ist konstruktionsbedingt am einfachsten realisierbar und in der Regel für eine ausreichend hohe Schadstoffreduzierung bereits ausreichend. In Sonderfällen kann allerdings vorgesehen sein, dass der Desinfektionsraum einen gewundenen Kanal (oder mehrere gewundene Kanäle) für den Gasstrom ausbildet, um die Verweildauer des Gases in dem Desinfektionsraum weiter zu erhöhen.

In einer vorteilhaften Weiterbildung der Erfindung kann vorgesehen sein, dass das flüssige Oxidationsmittel Wasserstoffperoxid (H₂O₂) ist oder Wasserstoffperoxid aufweist.

Wasserstoffperoxid weist bereits für sich genommen eine hohe desinfizierende Wirkung auf, die zu einer befriedigenden Reinigung des Gases von den Schadstoffen führen kann.

Insbesondere aber in Kombination mit der ionisierenden Strahlung, vor allem der UV-C-Strahlung (besonders bevorzugt einer UV-C-Strahlung mit einer Wellenlänge von 170 nm oder weniger), kann eine besonders starke Reinigungswirkung erreicht werden, die sich für einen hohen Gasdurchsatz eignet. Durch die Wirkung der UV-C-Strahlung auf das Wasserstoffperoxid werden Hydroxylradikale (OH-Radikale) erzeugt, deren oxidative Wirkung die des Wasserstoffperoxids noch übersteigen. Die Hydroxylradikale sind bei der Reduzierung der Schadstoffbelastung innerhalb des Gases schließlich besonders effektiv.

Durch die UV-C-Bestrahlung des Wasserstoffperoxids bzw. des Oxidationsmittels kann insbesondere auch sichergestellt werden, dass das Oxidationsmittel bzw. Wasserstoffperoxid aus dem Gasstrom ausreichend entfernt und in Hydroxylradikale umgesetzt wird. Die Hydroxylradikale sind vergleichsweise kurzlebig und treten demnach in der Regel nicht oder zumindest in einem nicht schädlichen Maße aus der Vorrichtung aus.

Das Wasserstoffperoxid kann in seiner reinen Form oder in Form eines Gemischs, vorzugsweise verdünnt mit Wasser oder Alkohol (beispielsweise Ethanol und/oder 1-Propanol und/oder 2-Propanol), vorgesehen sein. Besonders bevorzugt ist eine Wasserstoffperoxid-Wasser-Lösung mit einer Konzentration von 1% bis 20% Wasserstoffperoxid, besonders bevorzugt 2% bis 10% Wasserstoffperoxid, weiter bevorzugt 3% bis 7% Wasserstoffperoxid, beispielsweise 5% Wasserstoffperoxid, vorgesehen.

Alternativ oder zusätzlich zu Wasserstoffperoxid können beispielsweise auch Peroxycarbonsäuren ("Persäuren") als Oxidationsmittel vorgesehen sein. Unter anderem kann Peroxyessigsäure, Peroktansäure, Pernonansäure und e-Phthalimid-Peroxo-Hexansäure oder ein Gemisch aus mehreren Peroxycarbonsäuren vorgesehen sein. Auch die vorstehend aufgelisteten Oxidationsmittel vermögen in Kombination mit der ionisierenden Strahlung, insbesondere der UV-C-Strahlung, Hydroxylradikale zu bilden.

Vorzugsweise weist der Desinfektionsraum ein erstes, unteres Ende und ein zweites, oberes Ende auf. Das untere Ende des Desinfektionsraums kann auch als "Boden" des Desinfektionsraums bezeichnet werden.

In einer vorteilhaften Weiterbildung der Erfindung kann vorgesehen sein, dass das Flüssigkeitsreservoir an dem unteren Ende des Desinfektionsraums ausgebildet wird.

Diese Variante hat sich aus Konstruktionsgründen als besonders vorteilhaft erwiesen. Das untere Ende bzw. der Boden des Desinfektionsraums kann entsprechend abgedichtet sein, so dass das Oxidationsmittel nicht entweicht.

Das Flüssigkeitsreservoir, insbesondere das untere Ende des Desinfektionsraums, kann beispielsweise bis zu 10 cm mit dem Oxidationsmittel gefüllt sein, beispielsweise auch nur bis zu 5 cm oder nur bis zu 2 cm oder weniger.

Grundsätzlich kann auch vorgesehen sein, dass das Flüssigkeitsreservoir als separater Behälter in dem Desinfektionsraum ausgebildet ist oder sogar außerhalb des Desinfektionsraums ausgebildet ist. Das kapillare Material der Aerosolerzeugungsanordnung kann sich somit auch aus dem Desinfektionsraum hinaus bis in das separate Flüssigkeitsreservoir erstrecken.

In einer Weiterbildung kann vorgesehen sein, dass die Vorrichtung einen Flüssigkeitstank für das flüssige Oxidationsmittel aufweist, der mit dem Flüssigkeitsreservoir fluidisch verbunden ist, um dem Flüssigkeitsreservoir bedarfsweise weiteres Oxidationsmittel zuzuführen.

Bei dem Flüssigkeitstank kann es sich um ein in der Vorrichtung fest integriertes, vorzugsweise nachfüllbares Behältnis (optional mit einem verschließbaren Deckel, einer Einfüllhilfe und/oder einer Dosierhilfe) und/oder um eine Kartusche / Patrone handeln, die der Benutzer der Vorrichtung bedarfsweise austauschen kann.

In einer Ausgestaltung der Erfindung kann eine Pumpe, beispielsweise eine Dosierpumpe, vorgesehen sein, um dem Flüssigkeitsreservoir aus dem Flüssigkeitstank bedarfsweise weiteres Oxidationsmittel zuzuführen.

Grundsätzlich kann eine beliebige Pumpenart eingesetzt werden, beispielsweise eine Strömungspumpe, eine Verdrängerpumpe oder eine sonstige Pumpe, beispielsweise auch eine Schlauchpumpe ("Schlauchquetschpumpe"). Die Pumpe ist vorzugsweise elektronisch ansteuerbar, um die dem Flüssigkeitsreservoir zuzuführende Oxidationsmittelmenge elektronisch zu dosieren. Es kann ggf. aber auch eine mechanische Pumpe vorgesehen sein. Das Flüssigkeitsreservoir kann dann vorteilhaft als Auffangbehälter für abtropfende Flüssigkeit dienen, die anschließend wieder durch die Kapillarwirkung des kapillaren Materials aufgenommen wird.

Optional kann ein Flüssigkeitssensor vorgesehen sein, um die Flüssigkeitsmenge innerhalb des Flüssigkeitsreservoirs und/oder innerhalb des Flüssigkeitstanks zu erfassen (kontinuierlich oder zu diskreten Zeiten).

Der Flüssigkeitssensor kann insbesondere als Füllstandsensor ausgebildet sein, der bei Unterschreiten eines oder mehrerer Schwellenwerte die Zugabe weiteren Oxidationsmittels aus dem Flüssigkeitstank in das Flüssigkeitsreservoir veranlasst, beispielsweise durch eine elektrische Signalübermittlung an die Pumpe.

Optional kann bei einem niedrigen Füllstand des Flüssigkeitstanks und/oder des Flüssigkeitsreservoirs dem Benutzer der Vorrichtung die Notwendigkeit des Nachfüllens des Flüssigkeitstanks und/oder des Flüssigkeitsreservoirs kenntlich gemacht werden, vorzugsweise optisch (z. B. durch eine Anzeige in einem Display und/oder durch eine oder mehrere Leuchtdioden) oder akustisch, z. B. durch einen Akustik-Signalgeber, insbesondere einen Lautsprecher oder einen elektronischen Summer. Auch ein vollständig oder abschnittsweise transparenter Flüssigkeitstank kann vorgesehen sein, um dem Benutzer der Vorrichtung den Füllstand erkennbar anzuzeigen.

In einer Weiterbildung kann außerdem vorgesehen sein, dass die Vorrichtung einen in dem Gasstrom angeordneten mechanischen Filter und/oder Aktivkohlefilter aufweist.

Vorzugsweise ist der mechanische Filter und/oder der Aktivkohlefilter unmittelbar an dem Gaseinlass angeordnet, vorzugsweise hinter einem Lüftungsgitter des Gaseinlasses.

Vorzugsweise ist der mechanische Filter und/oder der Aktivkohlefilter zwischen dem Gaseinlass und dem Desinfektionsraum angeordnet, um dem Desinfektionsraum bereits einen partikelreduzierten Gasstrom zuzuführen.

Vorzugsweise ist der mechanische Filter und/oder der Aktivkohlefilter von dem Benutzer austauschbar. Es kann optional ein Betriebsstundenzähler vorgesehen sein, um dem Benutzer anhand der erfassten Betriebsstunden ggf. einen Austausch des mechanischen Filters und/oder des Aktivkohlefilters zu empfehlen.

Durch den mechanischen Filter und/oder den Aktivkohlefilter können größere Partikel, insbesondere auch anorganische Partikel, komfortabel entfernt werden.

In einer vorteilhaften Weiterbildung kann vorgesehen sein, dass die Vorrichtung wenigstens eine Strömungsmaschine zur Erzeugung des Gasstroms zwischen dem Gaseinlass und dem Gasauslass aufweist.

Vorzugsweise ist ein Ventilator bzw. Lüfter vorgesehen, insbesondere ein elektronisch steuer- bzw. regelbarer Lüfter. Die Betriebsspannung der Strömungsmaschine kann vorzugsweise pulsweitenmodulierbar sein.

Die wenigstens eine Strömungsmaschine kann im Bereich des Gaseinlasses angeordnet sein, um das Gas anzusaugen und den Gasstrom in Richtung auf den Desinfektionsraum wieder auszugeben. Die wenigstens eine Strömungsmaschine kann alternativ oder zusätzlich aber auch im Bereich des Gasauslasses angeordnet sein, um den Gasstrom aus dem Desinfektionsraum anzusaugen und aus dem Gasauslass auszugeben. Grundsätzlich kann sogar eine Anordnung vorgesehen sein, bei der die Strömungsmaschine innerhalb des Desinfektionsraums angeordnet ist, beispielsweise vor, innerhalb und/oder nach der Aerosolerzeugungsanordnung oder der Strahlungsquelle.

Grundsätzlich kann die Strömungsmaschine an einer beliebigen Position angeordnet sein, vorzugsweise seitlich des Desinfektionsraums, gegebenenfalls aber auch unterhalb oder oberhalb des Desinfektionsraums.

Die Strömungsmaschine kann eingerichtet und angeordnet sein, um das Gas von außerhalb der Vorrichtung anzusaugen und in die Vorrichtung zu drücken und/oder um das Gas aus der Vorrichtung anzusaugen und aus der Vorrichtung hinaus zu drücken.

Um den Gasstrom bzw. Volumendurchsatz zu vergrößern können vorzugsweise mehrere Strömungsmaschinen vorgesehen sein, beispielsweise zwei, drei, vier oder noch mehr Strömungsmaschinen, insbesondere in paralleler Anordnung nebeneinander.

An dieser Stelle sei vorsorglich erwähnt, dass die Vorrichtung nicht unbedingt eine Strömungsmaschine erfordert. Die Vorrichtung ist grundsätzlich auch in einem bereits bestehenden Gasstrom anordenbar, beispielsweise wenn der Gasstrom auf natürliche Ursache erzeugt wird (z. B. Wind bzw. natürliche Luftströmung) und/oder wenn der Gasstrom von einer anderen Einrichtung erzeugt wird, beispielsweise der nachfolgend noch genannten Basiseinheit - also insbesondere, wenn die Vorrichtung als Erweiterung einer bestehenden Einrichtung mit einer Strömungsmaschine und/oder in einer passiven Lüftungsanlage verwendet wird.

In einer Ausgestaltung der Erfindung kann vorgesehen sein, dass dem Gas vor dem Ausgeben durch den Gasauslass ein Duftstoff beigefügt wird, um das Wohlbefinden der Benutzer der Vorrichtung positiv zu beeinflussen. Vorzugsweise wird der Duftstoff unmittelbar vor der Ausgabe des Gases beigefügt, damit dieser durch die vorgeschlagenen Reinigungsprozesse nicht wieder abgebaut wird. Der Duftstoff kann gegebenenfalls aber auch mit dem flüssigen Oxidationsmittel vermischt und damit über dieselben Verteilungsprozesse in dem Gas verteilt werden, wie das Oxidationsmittel. Diese Variante eignet sich vorzugsweise aber nur bei einem gegenüber dem Oxidationsmittel robusten und damit nicht oder nur wenig abbaubaren Duftstoff.

In einer Weiterbildung der Erfindung kann vorgesehen sein, dass die Vorrichtung wenigstens ein zwischen dem Gaseinlass und dem Gasauslass angeordnetes Schallabsorberelement aufweist. Das Schallabsorberelement ist vorzugsweise innerhalb des Desinfektionsraums angeordnet.

Bei dem Schallabsorberelement kann es sich beispielsweise um ein schallabsorbierendes Gewebe, eine Matte, einen Schaumstoff, ein Vlies oder ein sonstiges schallabsorbierendes Material handeln.

Insbesondere kann vorgesehen sein, dass das Schallabsorberelement derart in dem Gasstrom positioniert ist, dass der Gasstrom gegen das Schallabsorberelement strömt und von dem Schallabsorberelement abgelenkt wird. Das Schallabsorberelement kann beispielsweise winklig zu dem Gasstrom ausgerichteten sein, und daher eine Prallfläche für den Gasstrom bilden. Dabei kann das Schallabsorberelement selbst plattenförmig in dem Gasstrom positioniert oder beispielsweise an einer abgewinkelten bzw. abknickenden Innenwandung des Strömungskanals angeordnet sein. Auch eine keilförmige Anordnung zumindest eines Schallabsorberelements kann vorgesehen sein.

Alternativ oder zusätzlich kann vorgesehen sein, dass das Schallabsorberelement angrenzend an eine Innenwandung der Vorrichtung verläuft, so dass der Gasstrom lateral an dem Schallabsorberelement entlangzuströmen vermag.

In einer Weiterbildung kann vorgesehen sein, dass das wenigstens eine Schallabsorberelement zumindest abschnittsweise in einer Rückhalteeinrichtung aufgenommen ist.

Die Rückhalteeinrichtung kann eingerichtet sein, das Schallabsorberelement innerhalb der Vorrichtung zu positionieren und auszurichten. Die Rückhalteeinrichtung kann hierzu beispielsweise innerhalb der Vorrichtung formschlüssig, kraftschlüssig und/oder stoffschlüssig befestigt sein, beispielsweise an einer Innenwandung des Desinfektionsraums.

Das Schallabsorberelement kann gegebenenfalls innerhalb der Rückhalteeinrichtung austauschbar sein bzw. aus der Rückhalteeinrichtung entnehmbar sein.

Die Rückhalteeinrichtung kann insbesondere aus einer doppelwandigen Einheit ausgebildet sein, die innerhalb der luftführenden Elemente oder innerhalb eines Luftkanals der Vorrichtung befestigt ist. Zwischen den Wandungen der Rückhalteeinrichtung kann das Schallabsorberelement eingebracht sein. Das Schallabsorberelement kann auch ein Bestandteil der Innenwandung der luftführenden Elemente bzw. des eines Luftkanals sein.

Es kann vorgesehen sein, dass die Rückhalteeinrichtung eine dem Gasstrom zugewandte Gitterfläche ausbildet, hinter der das Schallabsorberelement angeordnet ist.

Unter einer "Gitterfläche" ist vorliegend eine Fläche mit regelmäßig oder unregelmäßig verteilten Öffnungen zu verstehen, um dem Gasstrom das Schallabsorberelement durch die Öffnungen hindurch zu präsentieren, so dass das Schallabsorberelement die von dem Gasstrom erzeugte Schallenergie entsprechend vermindern kann. Die Öffnungen sind vorzugsweise rund, können aber im Grunde eine beliebige Form aufweisen.

In einer Weiterbildung der Erfindung kann optional vorgesehen sein, dass zumindest ein Teil des kapillaren Materials der Aerosolerzeugungsanordnung durch wenigstens eines der Schallabsorberelemente gebildet ist.

Das Schallabsorberelement kann also mit dem flüssigen Oxidationsmittel getränkt oder benetzt sein und dadurch das reinigende Aerosol in das vorbeistreichende, zu reinigende Gas einbringen. Das Schallabsorberelement kann ergänzend zu dem vorstehend bereits genannten kapillaren Material zu Einsatz kommen oder dieses ersetzen. Das schallabsorbierende Gewebe des Schallabsorberelements kann vorzugsweise kapillare Eigenschaften aufweisen.

Die vorstehend für das kapillare Material vorgeschlagenen Merkmale können daher auch für das Schallabsorberelement entsprechend gelten. Beispielsweise kann das Schallabsorberelement aus einem porösen, anorganischen Material ausgebildet sein, vorzugsweise aus einem textilen Glasfasergebilde. Das Schallabsorberelement kann sich bis in das Flüssigkeitsreservoir erstrecken und/oder von dem flüssigen Oxidationsmittel auf andere Weise benetzt werden.

Es kann aber auch vorgesehen sein, dass das Schallabsorberelement von dem kapillaren Material unabhängig ist und nicht als kapillares Material verwendet wird.

Die Erfindung betrifft auch ein System zur Beeinflussung eines Gasstroms, aufweisend eine Basiseinheit und eine Vorrichtung gemäß den vorstehenden und nachfolgenden Ausführungen, wobei die Vorrichtung als Erweiterung der Basiseinheit derart fluidisch mit der Basiseinheit verbunden ist, dass ein von der Basiseinheit beeinflusster bzw. erzeugter Gasstrom durch den Gaseinlass der Vorrichtung geleitet wird, um in den Desinfektionsraum zu gelangen und um aus dem Gasauslass schließlich wieder auszutreten.

Die Basiseinheit weist vorzugsweise eine Basiseinheit mit wenigstens einer Strömungsmaschine zur Erzeugung des Gasstroms auf. In diesem Fall wird also beispielsweise eine Luftbewegung durch einen vorhandenen Ventilator der zu erweiternden Basiseinheit erzeugt. Die Basiseinheit kann allerdings auch eine passive Einheit sein, die einen bereits bestehenden Gasstrom führt.

Die Vorrichtung kann in Strömungsrichtung vor, innerhalb oder hinter der Basiseinheit angeordnet sein, beispielsweise vor, innerhalb oder hinter einem Strömungskanal der Basiseinheit.

Die erfindungsgemäße Vorrichtung kann, wie vorstehend bereits erwähnt, auch ohne eingebauten Motor bzw. ohne integrierte Strömungsmaschine eingesetzt werden, insbesondere als Anbau- oder Ergänzungsmodul zur Erweiterung einer bestehenden Basiseinheit.

Die Nachrüstung kann sowohl an ortsfesten Basiseinheiten (wie z. B. Lüftungssysteme von Gebäuden) als auch an mobilen Basiseinheiten (wie z. B. Maschinen oder Fahrzeuge) erfolgen.

In Kombination mit dem Schallabsorberelement ist es optional möglich, neben der Schadstoffbelastung des Gases außerdem die Schallbelastung durch die Basiseinheit zu reduzieren.

Bei der Basiseinheit kann es sich um eine beliebige Einheit handeln, die einen Gasstrom erzeugen, umleiten und/oder manipulieren kann.

Bei der Basiseinheit kann es sich beispielsweise um einen Staubsauger, insbesondere um einen Industriesauger handeln, wobei die Vorrichtung insbesondere mit dem Staubsauger bzw. Industriesauger verbunden ist, um die Schadstoffe in der an die Umgebung wieder abgegebenen Abluft zu reduzieren.

Bei der Basiseinheit kann es sich beispielsweise auch um eine Abluftanlage handeln, wie zum Beispiel eine Dunstabzugshaube (Abluft oder Umluft) zum Absaugen und/oder Filtern von beim Kochen erzeugtem Dunst, oder um ein Abluftsystem in einem Chemielabor.

Bei der Basiseinheit kann es sich beispielsweise auch um eine Belüftungsanlage handeln oder um ein Umluftsystem.

Die Erfindung betrifft auch ein Verfahren zur Reduzierung einer Schadstoffbelastung in einem Gas gemäß Anspruch 13.

Die Erfindung eignet sich vorteilhaft zur Desinfektion bis Sterilisation von Gasen oder Gasgemischen, insbesondere von (Raum)Luft, und grundsätzlich zur Filtrierung bzw. Eliminierung von Schadstoffen aller Art, insbesondere von Staub und mikrobiologischen Krankheitserregern.

Die Erfindung kann insbesondere vorteilhaft zur Reinigung der Raumluft in Privathaushalten, Gewerbebetrieben, der Industrie oder in medizinischen Einrichtungen eingesetzt werden. Die Erfindung eignet sich beispielsweise zum Einsatz in Krankenhäusern, Altenheimen, Hotels, in der Gastronomie, in öffentlichen Einrichtungen und in Toiletten. Ein vorteilhafter Einsatz kann auch die Behandlung von Allergien betreffen, insbesondere die Reduzierung der Belastung der Luft mit Hausstaubmilben, Tierhaaren und dergleichen.

Die erfindungsgemäße Vorrichtung bzw. das erfindungsgemäße Verfahren kann zur Sicherstellung der Hygiene in Haushalt, Industrie, Pharmazie, Gewerbe und Medizin dienen.

Die Erfindung betrifft auch ein Computerprogrammprodukt mit Programmcodemitteln, um ein Verfahren gemäß den vorstehenden und nachfolgenden Ausführungen durchzuführen, wenn das Programm auf einer Steuereinrichtung einer Vorrichtung zur Reduzierung einer Schadstoffbelastung in einem Gas ausgeführt wird.

Die Steuereinrichtung kann als Mikroprozessor ausgebildet sein. Anstelle eines Mikroprozessors kann auch eine beliebige weitere Einrichtung zur Implementierung der Steuereinrichtung vorgesehen sein, beispielsweise eine oder mehrere Anordnungen diskreter elektrischer Bauteile auf einer Leiterplatte, eine speicherprogrammierbare Steuerung (SPS), eine anwendungsspezifische integrierte Schaltung (ASIC) oder eine sonstige programmierbare Schaltung, beispielsweise auch ein Field Programmable Gate Array (FPGA), eine programmierbare logische Anordnung (PLA) und/oder ein handelsüblicher Computer.

Die Erfindung betrifft außerdem eine vorteilhafte Verwendung einer vorstehend und nachfolgend beschriebenen Vorrichtung als mobiles (vorzugsweise freistehendes) Luftreinigungsgerät für Privathaushalte, Gewerbebetriebe, die Industrie oder für medizinische Einrichtungen.

Die Erfindung betrifft ferner eine vorteilhafte Verwendung einer vorstehend und nachfolgend beschriebenen Vorrichtung zur Erweiterung einer herkömmlichen Lüftungsanlage, wobei die Vorrichtung beispielsweise innerhalb eines Luftkanals angeordnet sein kann.

Merkmale, die im Zusammenhang mit dem erfindungsgemäßen Verfahren oder dem System beschrieben wurden, sind selbstverständlich auch für die erfindungsgemäße Vorrichtung vorteilhaft umsetzbar - und umgekehrt. Ferner können Vorteile, die im Zusammenhang mit dem erfindungsgemäßen Verfahren oder dem System genannt wurden, auch auf die erfindungsgemäße Vorrichtung bezogen verstanden werden - und umgekehrt.

Ergänzend sei darauf hingewiesen, dass Begriffe wie "umfassend", "aufweisend" oder "mit" keine anderen Merkmale oder Schritte ausschließen. Ferner schließen Begriffe wie "ein" oder "das", die auf eine Einzahl von Schritten oder Merkmalen hinweisen, keine Mehrzahl von Merkmalen oder Schritten aus - und umgekehrt.

In einer puristischen Ausführungsform der Erfindung kann allerdings auch vorgesehen sein, dass die in der Erfindung mit den Begriffen "umfassend", "aufweisend" oder "mit" eingeführten Merkmale abschließend aufgezählt sind. Dementsprechend kann eine oder können mehrere Aufzählungen von Merkmalen im Rahmen der Erfindung als abgeschlossen betrachtet werden, beispielsweise jeweils für jeden Anspruch betrachtet. Die Erfindung kann beispielsweise ausschließlich aus den in Anspruch 1 genannten Merkmalen bestehen. Beispielsweise kann vorgesehen sein, dass die Aerosolerzeugungsanordnung ausschließlich aus dem kapillaren Material besteht.

Es sei erwähnt, dass Bezeichnungen wie "erstes" oder "zweites" etc. vornehmlich aus Gründen der Unterscheidbarkeit von jeweiligen Vorrichtungs- oder Verfahrensmerkmalen verwendet werden und nicht unbedingt andeuten sollen, dass sich Merkmale gegenseitig bedingen oder miteinander in Beziehung stehen.

Ferner sei betont, dass die vorliegend beschriebenen Werte und Parameter Abweichungen oder Schwankungen von ±10% oder weniger, vorzugsweise ±5% oder weniger, weiter bevorzugt ±1% oder weniger, und ganz besonders bevorzugt ±0,1% oder weniger des jeweils benannten Wertes bzw. Parameters mit einschließen, sofern diese Abweichungen bei der Umsetzung der Erfindung in der Praxis nicht ausgeschlossen sind. Die Angabe von Bereichen durch Anfangs- und Endwerte umfasst auch all diejenigen Werte und Bruchteile, die von dem jeweils benannten Bereich eingeschlossen sind, insbesondere die Anfangs- und Endwerte und einen jeweiligen Mittelwert.

Die Erfindung betrifft auch eine von Patentanspruch 1 unabhängige Vorrichtung zur Behandlung eines Gases, aufweisend einen Desinfektionsraum mit einer Aerosolerzeugungsanordnung, die ausgebildet ist, um aus einem flüssigen Oxidationsmittel und dem Gas ein Aerosol zu erzeugen. Die Patentansprüche und die vorstehend und nachfolgend beschriebenen Merkmale betreffen vorteilhafte Ausführungsformen und Varianten dieser Vorrichtung.

Nachfolgend werden Ausführungsbeispiele der Erfindung anhand der Zeichnung näher beschrieben.

Die Figuren zeigen jeweils bevorzugte Ausführungsbeispiele, in denen einzelne Merkmale der vorliegenden Erfindung in Kombination miteinander dargestellt sind. Merkmale eines Ausführungsbeispiels sind auch losgelöst von den anderen Merkmalen des gleichen Ausführungsbeispiels umsetzbar und können dementsprechend von einem Fachmann ohne Weiteres zu weiteren sinnvollen Kombinationen und Unterkombinationen mit Merkmalen anderer Ausführungsbeispiele verbunden werden.

In den Figuren sind funktionsgleiche Elemente mit denselben Bezugszeichen versehen.

Es zeigen schematisch:
- Figur 1: eine Vorrichtung zur Reduzierung einer Schadstoffbelastung in einem Gas gemäß einem ersten Ausführungsbeispiel;
- Figur 2: eine Vorrichtung zur Reduzierung einer Schadstoffbelastung in einem Gas gemäß einem zweiten Ausführungsbeispiel in einer perspektivischen Darstellung;
- Figur 3: die Vorrichtung der Figur 2 in einer perspektivischen Schnittdarstellung;
- Figur 4: die Vorrichtung der Figur 2 in einer weiteren Schnittdarstellung;
- Figur 5: einen Ausschnitt einer Vorrichtung zur Reduzierung einer Schadstoffbelastung in einem Gas gemäß einem weiteren Ausführungsbeispiel der Erfindung in einer Schnittdarstellung;
- Figur 6: die Vorrichtung der Figur 5 mit einer geschnittenen Einzeldarstellung des Desinfektionsraums gemäß Schnittlinie VI in Figur 5;
- Figur 7: eine Variante des Desinfektionsraums mit Luftleitlamellen;
- Figur 8: einen Abschnitt zwischen dem Gaseinlass und dem Gasauslass einer erfindungsgemäßen Vorrichtung mit einem Schallabsorberelement, gemäß einer ersten Variante;
- Figur 9: einen Abschnitt zwischen dem Gaseinlass und dem Gasauslass einer erfindungsgemäßen Vorrichtung mit einem Schallabsorberelement, gemäß einer zweiten Variante;
- Figur 10: einen Abschnitt zwischen dem Gaseinlass und dem Gasauslass einer erfindungsgemäßen Vorrichtung mit einem Schallabsorberelement, gemäß einer dritten Variante;
- Figur 11: einen Abschnitt zwischen dem Gaseinlass und dem Gasauslass einer erfindungsgemäßen Vorrichtung mit einem Schallabsorberelement, gemäß einer vierten Variante; und
- Figur 12: ein erfindungsgemäßes System zur Beeinflussung eines Gasstroms mit einer als Abluftanlage ausgebildeten Basiseinheit und einer als Erweiterung in dem Abluftkanal der Abluftanlage angeordneten Vorrichtung zur Reduzierung der Schadstoffbelastung in dem durch den Abluftkanal strömenden Gas.

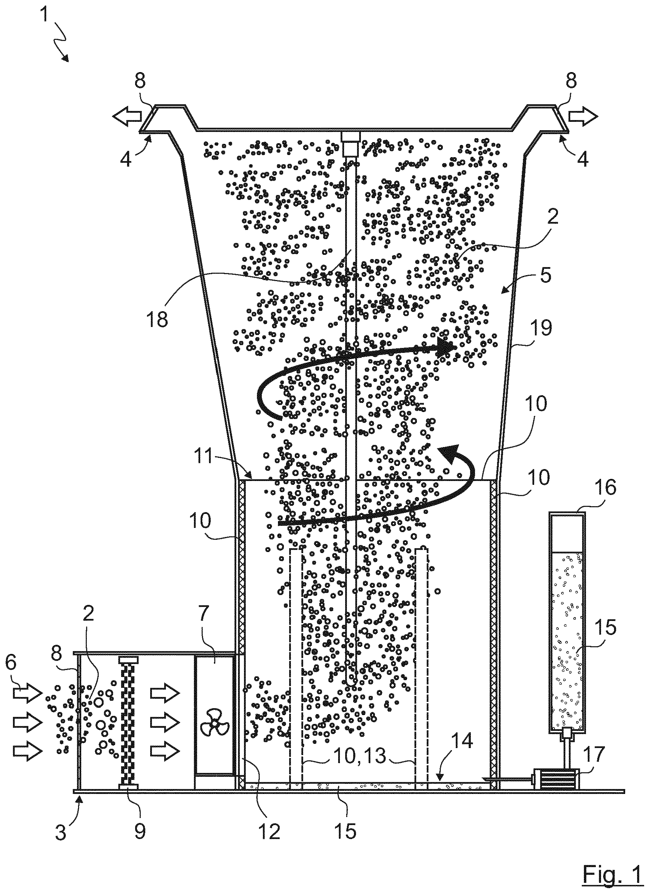

Figur 1 zeigt in schematisierter Darstellung eine Vorrichtung 1 zur Reduzierung einer Schadstoffbelastung in einem Gas 2.

Die in dem Ausführungsbeispiel gezeigten Vorrichtungen 1 sind, ohne dass die Erfindung auf diesen Einsatz beschränkt zu verstehen ist, zur Desinfektion oder Sterilisation von (Raum)Luft 2 vorgesehen. Insbesondere können mittels der erfindungsgemäßen Vorrichtung 1 Viren, Bakterien, Sporen oder sonstige organische und anorganische Schadstoffe aus der Luft 2 entfernt oder zumindest unschädlich gemacht werden.

Die in Figur 1 dargestellte Vorrichtung 1 weist einen Gaseinlass 3 und zwei Gasauslässe 4 auf. Zwischen dem Gaseinlass 3 und den Gasauslässen 4 ist ein Desinfektionsraum 5 angeordnet, der von einem von dem Gaseinlass 3 zu den Gasauslässen 4 verlaufenden Gasstrom 6 durchströmt wird. Der Gasstrom 6 ist in Figur 1 durch verschiedene Pfeile angedeutet.

Zur Erzeugung des Gasstroms 6 weisen die Vorrichtungen 1 des Ausführungsbeispiels entsprechende Strömungsmaschinen 7 auf. Dabei kann es sich insbesondere um einen elektronisch ansteuerbaren Ventilator 7 handeln. Die Strömungsmaschine(n) 7 kann bzw. können grundsätzlich an einer beliebigen Stelle innerhalb der Vorrichtung 1 angeordnet sein, vorzugsweise aber im Bereich des Gaseinlasses 3, wie in den Ausführungsbeispielen gezeigt.

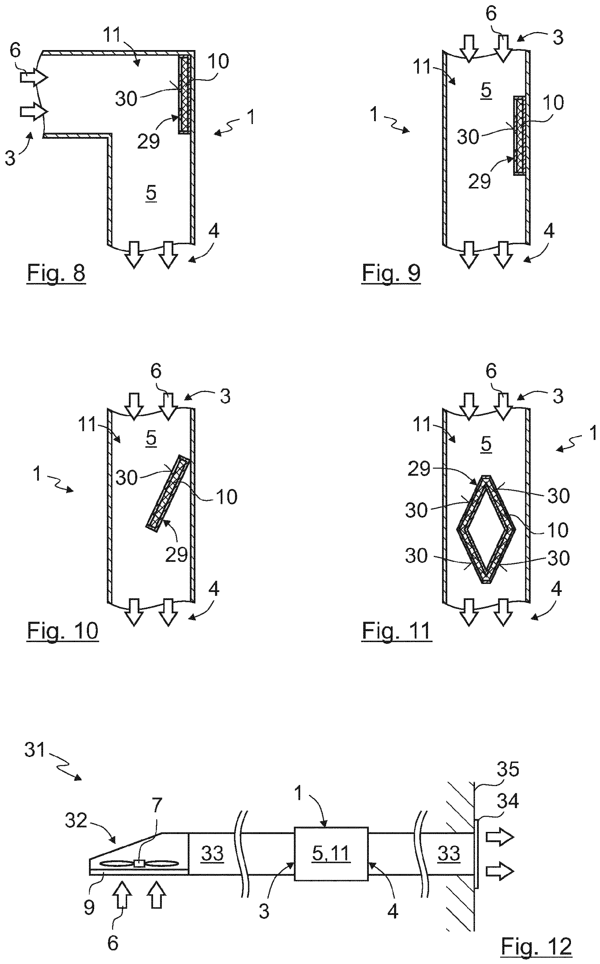

Grundsätzlich ist es nicht unbedingt erforderlich, dass die Vorrichtung 1 überhaupt eine Strömungsmaschine 7 aufweist. Die Vorrichtung 1 kann beispielsweise auch derart in einem bestehenden Gasstrom 6 angeordnet sein, dass dieser den Desinfektionsraum 5 durchströmt. Die Vorrichtung 1 kann hierzu beispielsweise in eine bestehende Lüftungsanlage integriert werden (beispielsweise in einen Lüftungsschacht einer Belüftungs- oder Klimaanlage), insbesondere wie dies nachfolgend im Rahmen der Figur 12 noch näher beschrieben wird.

Optional kann die Vorrichtung 1 an dem Gaseinlass 3 und/oder an dem Gasauslass 4 Lüftungsgitter 8 aufweisen um das Eindringen von Gegenständen in die Vorrichtung 1 zu blockieren, um einen Schutz gegen Beschädigung oder Manipulation für die Vorrichtung 1 bereitzustellen.

Ferner können innerhalb der Vorrichtung 1 diverse mechanische Filter und/oder Aktivkohlefilter 9 in dem Gasstrom 6 angeordnet werden, die gröbere Partikel zu entfernen vermögen und/oder bereits eine erste oder zusätzliche Reinigung durchführen. Gegebenenfalls kann auch die Verwendung von Schwebstofffiltern vorgesehen kann - dies ist allerdings nicht bevorzugt, da diese nur einen beschränkten Gasdurchsatz ermöglichen.

Optional vorhandene mechanische Filter und/oder Aktivkohlefilter 9 sind vorzugsweise im Bereich des Gaseinlasses 3 angeordnet, insbesondere hinter einem Lüftungsgitter 8, und vorzugsweise noch vor der wenigstens einen Strömungsmaschine 7.

Die Vorrichtung 1 weist ferner eine ein kapillares Material 10 aufweisende Aerosolerzeugungsanordnung 11 innerhalb des Desinfektionsraums 5 auf. Die Aerosolerzeugungsanordnung 11 ist von dem Gasstrom 6 durchströmbar. Das kapillare Material 10, bei dem es sich vorzugsweise um ein poröses, anorganisches Material handelt, insbesondere ein textiles Glasfasergebilde, beispielsweise ein Glasfasergeflecht oder eine Glasfasermatte, kleidet vorzugsweise wenigstens eine der Innenwandungen des Desinfektionsraums 5 teilweise aus. In den Innenwandungen vorhandenen Ausnehmungen 12 zum Gaseinlass und/oder Gasauslass können ausgespart sein.

Grundsätzlich kann das kapillare Material 10 der Aerosolerzeugungsanordnung 11 allerdings beliebig innerhalb des Desinfektionsraums 5 verteilt angeordnet sein, insbesondere derart, dass das kapillare Material 10 dem vorbeiströmenden Gas 2 möglichst viel Oberfläche präsentiert. Beispielsweise kann die Aerosolerzeugungsanordnung 11 auch wenigstens einen aus dem kapillaren Material 10 gebildeten länglichen Körper 13 aufweisen (in Figur 1 strichliniert dargestellt), der sich durch den Desinfektionsraum 5 erstreckt. Vorzugsweise sind mehrere längliche Körper 13 vorgesehen, die beispielsweise an einem ihrer Enden gemeinsam befestigt sind (nicht dargestellt).

Es ist vorgesehen, dass sich das kapillare Material 10 bis in ein Flüssigkeitsreservoir 14 erstreckt, in dem ein flüssiges Oxidationsmittel 15 enthalten ist. In den Ausführungsbeispielen ist das Flüssigkeitsreservoir 14 an dem unteren Ende des Desinfektionsraums 5 ausgebildet, der entsprechend abgedichtet ist. Durch die kapillaren Eigenschaften des kapillaren Materials 10 wird das Oxidationsmittel 15 schließlich aufgesaugt und steigt in dem kapillaren Material 10 nach oben, bis die Oberfläche des kapillaren Materials 10 vollständig von dem Oxidationsmittel 15 benetzt ist. Das vorbeiströmende Gas 2 nimmt somit in Abhängigkeit der bereitgestellten Oberfläche eine entsprechende Menge des Oxidationsmittels 15 auf.

Vorzugsweise ist das flüssige Oxidationsmittel 15 Wasserstoffperoxid oder weist Wasserstoffperoxid auf. Insbesondere kann ein Wasserstoffperoxid-Wasser-Gemisch vorgesehen sein, beispielsweise mit einer Konzentration von 5% Wasserstoffperoxid.

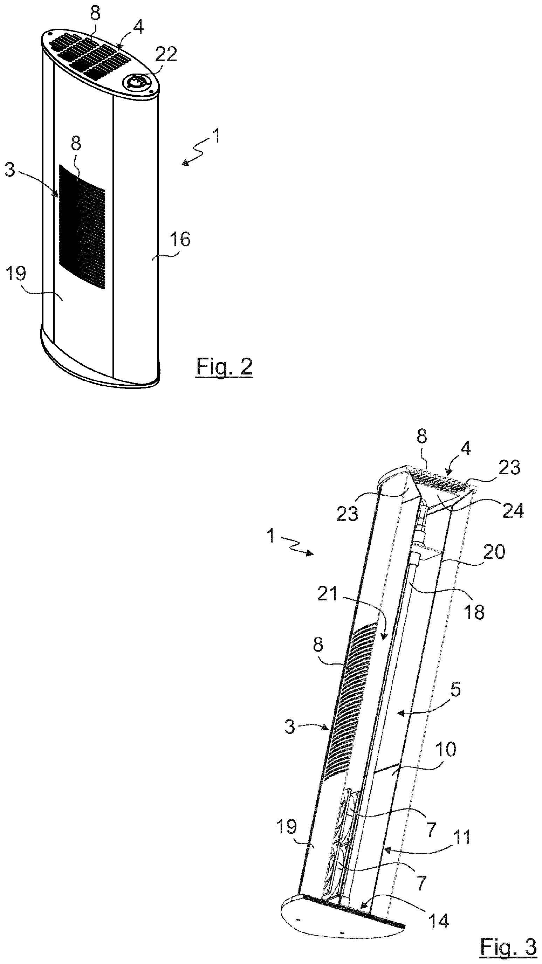

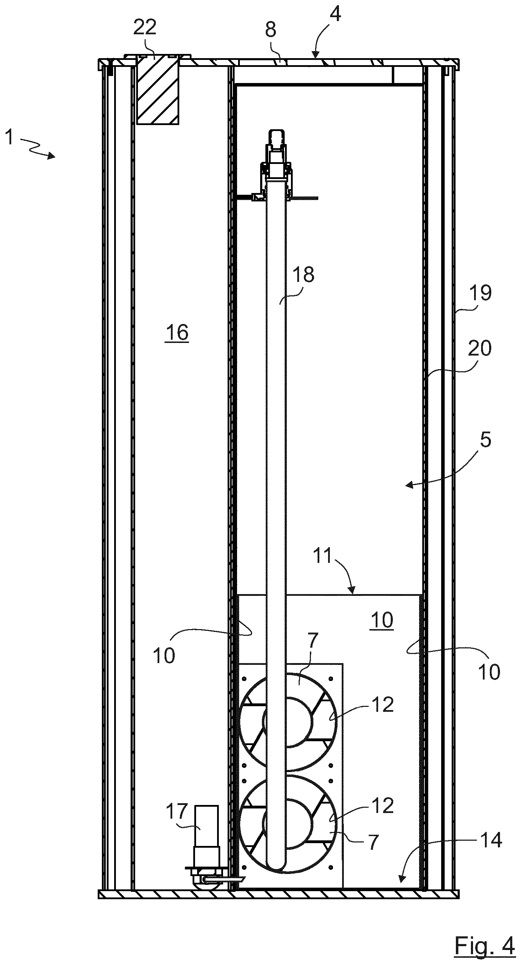

Optional kann ein Flüssigkeitstank 16 für das flüssige Oxidationsmittel 15 vorgesehen sein, der mit dem Flüssigkeitsreservoir 14 fluidisch verbunden ist. Bei dem Flüssigkeitstank 16 kann es sich beispielsweise um eine Kartusche handeln, die der Benutzer der Vorrichtung 1 leicht austauschen kann. Eine Kartuschenlösung ist in Figur 1 angedeutet; einen auffüllbaren Flüssigkeitstank 16 zeigt hingegen das nachfolgend noch beschriebenen Ausführungsbeispiel der Figuren 2 bis 4.

Um dem Flüssigkeitsreservoir 14 bedarfsweise weiteres Oxidationsmittel 15 aus dem Flüssigkeitstank 16 zuzuführen, kann eine Pumpe 17 vorgesehen sein, beispielsweise eine Schlauchquetschpumpe oder eine sonstige vorteilhafte Dosierpumpe. Ergänzend kann optional ein Flüssigkeitssensor vorgesehen sein (nicht dargestellt), der das Vorhandensein von Flüssigkeit in dem Flüssigkeitsreservoir 14 und/oder einen Pegelstand der Flüssigkeit in dem Flüssigkeitsreservoir 14 erfasst und die Pumpe 17 gegebenenfalls veranlasst, weiteres Oxidationsmittel 15 zuzuführen.

Die fluidische Verbindung zwischen einem optionalen Flüssigkeitstank 16 und dem Flüssigkeitsreservoir 14 muss nicht unbedingt im Bereich des unteren Endes der Vorrichtung 1 vorgesehen sein, wie dies in den Ausführungsbeispielen angedeutet ist. Beispielsweise kann auch vorgesehen sein, dem Desinfektionsraum 5 das flüssige Oxidationsmittel 15 aus dem Flüssigkeitstank 16 ausgehend von einer höheren Höhenlage zuzuführen und das Oxidationsmittel 15 beispielsweise entlang des kapillaren Materials 10 hinunterlaufen zu lassen. Optional (aber nicht bevorzugt) kann auch ein ergänzendes Versprühen des Oxidationsmittels 15 innerhalb des Desinfektionsraums 5 vorgesehen sein, wobei sich überschüssiges Oxidationsmittel 15 in dem Flüssigkeitsreservoir 14 sammeln kann.

In den Ausführungsbeispielen ist vorgesehen, dass innerhalb des Desinfektionsraums 5 wenigstens eine Strahlungsquelle 18 zur Erzeugung einer ionisierenden Strahlung, insbesondere einer UV-C-Strahlung, vorgesehen ist. Bei der Strahlungsquelle 18 kann es sich insbesondere um eine UV-C-Röhre oder um eine oder mehrere UV-C-Leuchtdioden handeln.

Bereits UV-C-Strahlung für sich genommen bietet einen hervorragenden keimtötenden Effekt. Insbesondere aber in Kombination mit dem flüssigen Oxidationsmittel 15, insbesondere einem in dem Gasstrom 6 enthaltenen Aerosol des Oxidationsmittels 15, kann eine außergewöhnlich starke Desinfektionswirkung verursacht werden. Die UV-C-Strahlung kann insbesondere in Reaktion mit Wasserstoffperoxid (und/oder beispielsweise den vorstehend genannten Peroxycarbonsäuren) Hydroxylradikale erzeugen. Das Hydroxylradikal ist ein noch stärkeres Oxidationsmittel als Wasserstoffperoxid und kann auf hocheffektive Weise die Schadstoffbelastung innerhalb des Gases 2 reduzieren.

Vorzugsweise ist die Strahlungsquelle 18 derart ausgebildet und/oder angeordnet, dass die ionisierende Strahlung den größten Teil des Desinfektionsraums 5 ausstrahlt, vorzugsweise den Desinfektionsraum 5 vollständig ausstrahlt. Dabei kann außerdem vorgesehen sein, dass die Längserstreckung des Desinfektionsraums 5 zwischen dem Gaseinlass 3 und dem Gasauslass 4 größer ist als der Durchmesser des Desinfektionsraums 5. Vorzugsweise bildet der Desinfektionsraum 5 einen linear verlaufenden Kanal für den Gasstrom 6 aus. Gegebenenfalls kann der Desinfektionsraum 5 aber auch einen oder mehrere gewundene Kanäle für den Gasstrom 6 ausbilden.

Ziel der vorstehend genannten Maßnahme ist es insbesondere sicherzustellen, dass das Gas 2 eine möglichst große Verweildauer innerhalb des Desinfektionsraums 5 aufweist, wodurch die ionisierende Strahlung, das Wasserstoffperoxid und/oder die Hydroxylradikale möglichst viele Schadstoffe reduzieren, bei gleichzeitig hohem Durchsatz des Gases 2.

Es kann vorgesehen sein, dass die Strahlungsquelle 18 zumindest abschnittsweise innerhalb der Aerosolerzeugungsanordnung 11 angeordnet ist, wie in den Ausführungsbeispielen dargestellt. Grundsätzlich kann allerdings auch vorgesehen sein, dass die Strahlungsquelle 18 der Aerosolerzeugungsanordnung 11 in Strömungsrichtung betrachtet nachgeordnet ist.

Die Strahlungsquelle 18 muss nicht unbedingt zentral innerhalb des Desinfektionsraums 5 angeordnet sein, wie in den Ausführungsbeispielen dargestellt. Die Strahlungsquelle 18 kann beispielsweise auch angrenzend oder benachbart zu einer der Seitenwandungen des Desinfektionsraums 5 verlaufen. In diesem Fall sind vorzugsweise mehrere Strahlungsquellen 18 vorgesehen, beispielsweise zwei der dargestellten UV-C-Röhren 18. Grundsätzlich können beliebig viele Strahlungsquellen 18 beliebiger Bauweise innerhalb des Desinfektionsraums 5 verteilt angeordnet sein.

In den Figuren 2 bis 4 ist ein zweiten Ausführungsbeispiel der Vorrichtung 1 dargestellt. Die grundsätzlichen Merkmale der nachfolgenden Ausführungsbeispiele entsprechen dem bereits im Zusammenhang mit Figur 1 beschriebenen Ausführungsbeispiel, weshalb nachfolgend im Wesentlichen auf die Unterschiede zwischen den Ausführungsbeispielen eingegangen wird.

Keines der in dieser Beschreibung dargestellten Ausführungsbeispiele ist für die Erfindung einschränkend zu verstehen und der Fachmann kann die Merkmale der Ausführungsbeispiele beliebig miteinander kombinieren, sofern dies technisch nicht ausgeschlossen ist.

Die Vorrichtung 1 des zweiten Ausführungsbeispiels weist einen einzigen Gaseinlass 3 und einen einzigen Gasauslass 4 auf, die jeweils von einem Lüftungsgitter 8 abgedeckt sind. Der Gaseinlass 3 bzw. das Lüftungsgitter 8 des Gaseinlasses 3 ist in einem Außengehäuse 19 der Vorrichtung 1 angeordnet.

Die Vorrichtung 1 weist außerdem ein Innengehäuse 20 auf, das im Wesentlichen den Desinfektionsraum 5 abgrenzt. Zwischen Außengehäuse 19 und Innengehäuse 20 ist ein Spalt 21 zur Führung des Gasstroms 6 von dem Gaseinlass 3 bis zu einer Ausnehmung 12 in dem Innengehäuse 20 zu dem Desinfektionsraum 5 vorgesehen.

Der Versatz zwischen dem Lüftungsgitter 8 des Außengehäuses 19 und der Ausnehmung 12 zu dem Desinfektionsraum 5 kann vorteilhaft sein, um die UV-C-Strahlung abzuschirmen bzw. um einen direkten Sichtkontakt in den Desinfektionsraum 5 zu unterbinden.

Optional (in Figur 2 bis 4 nicht dargestellt) kann wiederum ein mechanischer Filter und/oder Aktivkohlefilter 9 vorgesehen sein, vorzugsweise hinter dem Lüftungsgitter 8 des Gaseinlasses 3, insbesondere austauschbar bzw. für den Benutzer zugänglich.

In dem Ausführungsbeispiel der Figuren 2 bis 4 sind zwei als Ventilatoren ausgebildete Strömungsmaschinen 7 in paralleler Anordnung vorgesehen, um das Gas 2 ausgehend von dem Gaseinlass 3 anzusaugen und durch den Desinfektionsraum 5 zu drücken, bis dieses schließlich wieder aus dem Gasauslass 4 entweicht.

Auch in dem Ausführungsbeispiel der Figuren 2 bis 4 ist das kapillare Material 10 der Aerosolerzeugungsanordnung 11 innenseitig an den Innenwandungen des Desinfektionsraums 5 befestigt und ragt in das sich im unteren Ende des Desinfektionsraums 5 befindliche Flüssigkeitsreservoir 14 hinein. Das flüssige Oxidationsmittel 15 ist in den Figuren 2 bis 4 nicht dargestellt.

Aus einem neben dem Desinfektionsraum 5 angeordneten Flüssigkeitstank 16 kann mittels einer Pumpe 17 optional weiteres Oxidationsmittel 15 dem Flüssigkeitsreservoir 14 zugeführt werden. Der Flüssigkeitstank 16 weist eine mit einem Deckel 22 verschließbare Zugangsöffnung auf, die es dem Benutzer ermöglicht, weiteres Oxidationsmittel 15 zuzuführen. Der Flüssigkeitstank 16 kann beispielsweise ein Volumen von einem Liter bis 20 Liter, vorzugsweise 2 Liter bis 15 Liter, besonders bevorzugt 5 Liter bis 10 Liter aufweisen.

Auch in dem Ausführungsbeispiel der Figuren 2 bis 4 ist eine UV-C-Röhre 18 vorgesehen. Wie bereits erwähnt, können grundsätzlich auch noch weitere UV-C-Strahlungsquellen 18 vorgesehen sein, wobei es auf die spezifische Bauart nicht ankommt.

Im Bereich des Gasauslasses 4 ist in dem Ausführungsbeispiel der Figuren 2 bis 4 ein Luftleitblech 23 vorgesehen (vgl. Figur 3), um die Luft 2 aus dem Desinfektionsraum 5 hinauszuleiten. Zwischen den Luftleitblechen 23 ist ein Abschirmblech 24 (vgl. ebenfalls Figur 3) zum Abschirmen der UV-C-Strahlung vorgesehen, beispielsweise ein Edelstahlblech.

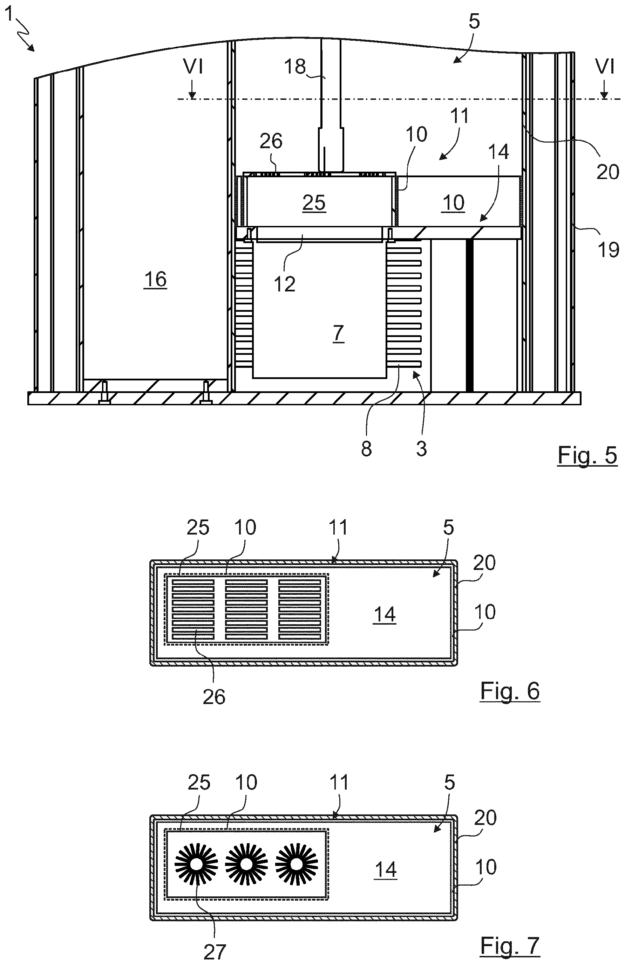

In den Figuren 5 und 6 ist ein drittes Ausführungsbeispiel der Vorrichtung 1 dargestellt, wobei Figur 6 eine Einzeldarstellung des Desinfektionsraums 5 in einer Schnittdarstellung gemäß Schnittlinie VI der Figur 5 zeigt; Figur 7 stellt eine weitere Variante des Desinfektionsraums 5 des dritten Ausführungsbeispiels dar. Das dritte Ausführungsbeispiel entspricht im Wesentlichen dem zweiten Ausführungsbeispiel, weist aber einige optionale, vorteilhafte Unterscheidungsmerkmale auf.

Die Vorrichtung 1 des dritten Ausführungsbeispiels weist einen im unteren Bereich der Vorrichtung 1 angeordneten Gaseinlass 3 auf, der wiederum von einem Lüftungsgitter 8 abgedeckt ist (vgl. Figur 5). Die Strömungsmaschine 7 ist nur schematisch als Black-Box angedeutet.

Das Innengehäuse 20 grenzt den Desinfektionsraum 5 ab. In einer Bodenplatte des Desinfektionsraums 5 ist eine Ausnehmung 12 vorgesehen, auf der ein Gaseinleitungsstutzen 25 aufgesetzt ist und in den Desinfektionsraum 5 hineinragt. Auch dieser Aufbau kann dazu beitragen, die UV-C-Strahlung der Strahlungsquelle 18 abzuschirmen bzw. um einen direkten Sichtkontakt in den Desinfektionsraum 5 ausgehend von dem Gaseinlass 3 zu unterbinden.

Das Flüssigkeitsreservoir 14 ist im unteren Bereich des Desinfektionsraums 5 ausgebildet. Durch den Gaseinleitungsstutzen 25 kann sichergestellt sein, dass das Oxidationsmittel 15 (in den Figuren 5 bis 7 nicht dargestellt) nicht aus dem Desinfektionsraum 5 durch die Ausnehmung 12 entweicht und beispielsweise in die Strömungsmaschine 7 eindringt und diese beschädigt.

Das kapillare Material 10 der Aerosolerzeugungsanordnung 11 kann wiederum innenseitig an den Innenwandungen des Desinfektionsraums 5 befestigt sein, wie in Figur 5 gut erkennbar. Alternativ oder zusätzlich kann auch vorgesehen sein, dass das kapillare Material 10 um die Außenwandungen des Gaseinleitungsstutzens 25 herum angeordnet ist bzw. außenseitig an dem Gaseinleitungsstutzen 25 befestigt ist (in den Figuren 6 und 7 strichliniert angedeutet).

Insbesondere wenn das kapillare Material 10 nur im unteren Bereich des Desinfektionsraums 5 angeordnet ist, kann es vorteilhaft sein, in diesem Bereich, beispielsweise angrenzend an die Ausnehmung 12 und/oder an den Gaseinleitungsstutzen 25, Verwirbelungen des Gases 2 zu erzeugen, um das Gas 2 möglichst effizient entlang der Oberfläche des kapillaren Materials vorbeizuführen und um die Verweildauer des Gases 2 im Bereich des kapillaren Materials 10 zu erhöhen. Hierzu können beispielsweise Leitbleche (nicht dargestellt), Strömungsgitter 26 (vgl. Figuren 5 und 6) und/oder Lamellenanordnungen 27 (vgl. Figur 7) vorgesehen sein.

In den Figuren 8 bis 11 sind verschiedene, beispielhafte Varianten eines Schallabsorberelements 28 dargestellt, das innerhalb der Vorrichtung 1 zwischen dem Lufteinlass 3 und dem Luftauslass 4 angeordnet sein kann, um die von dem Gasstrom 6 verursachten Schallemissionen zu verringern. Vorzugsweise ist das Schallabsorberelement 28 innerhalb des Desinfektionsraums 5 bzw. in der Aerosolerzeugungsanordnung 11 angeordnet. Das Schallabsorberelement 28 kann gegebenenfalls auch als Ersatz oder Ergänzung zu dem kapillaren Material 10 in der Aerosolerzeugungsanordnung 11 verwendet werden und mit dem flüssigen Oxidationsmittel 15 benetzt sein.

Das Schallabsorberelement 28 kann in einer Rückhalteeinrichtung 29 aufgenommen sein, die zumindest eine Gitterfläche 30 ausbildet, die dem Gasstrom 6 zugewandt ist, und hinter der das Schallabsorberelement 28 angeordnet ist. In den Varianten der Figuren 8 bis 10 ist jeweils nur eine Gitterfläche 30 vorgesehen; in der Variante der Figur 11 ist beispielhaft eine keilförmige Anordnung vorgesehen, bei der alle Außenflächen der Rückhalteeinrichtung 29 mit Gitterflächen 30 versehen sind. Das Schallabsorberelement 28 kann somit auf vorteilhafte Weise innerhalb der doppelwandigen Rückhalteeinrichtung 29 aufgenommen sein, und dennoch seine schallabsorbierende Wirkung entfalten.

Das Schallabsorberelement 28 kann in dem Gasstrom 6 positioniert sein, so dass der Gasstrom 6 gegen das Schallabsorberelement 28 strömt und von dem Schallabsorberelement 28 abgelenkt wird. Das Schallabsorberelement 28 kann beispielsweise eine Prallfläche bilden, wie in den Figuren 8, 10 und 11 dargestellt. Beispielsweise kann das Schallabsorberelement 28 an bzw. vor einer abknickenden Wandung des Strömungskanals (vgl. Figur 8) oder innerhalb des Strömungskanals (vgl. Figur 10 und 11) angeordnet sein.

Das Schallabsorberelement 28 kann allerdings auch angrenzend an eine Innenwandung der Vorrichtung 1 verlaufende angeordnet sein, so dass der Gasstrom 6 lateral an dem Schallabsorberelement 28 entlangzuströmen vermag, wie dies in Figur 9 angedeutet ist.

Schließlich ist in Figur 12 noch ein System 31 zur Beeinflussung eines Gasstroms 6 beispielhaft dargestellt, in dem sich die vorstehend beschriebene Vorrichtung 1 als Erweiterung einer Basiseinheit 32 vorteilhaft einsetzen lässt. Die Basiseinheit 32 ist beispielhaft als Dunstabzugshaube dargestellt. Es kann sich aber grundsätzlich um ein beliebiges Be- und Entlüftungssystem handeln, auch um ein Umluftsystem, und beispielsweise außerdem auch um ein bestehendes (Luft)reinigungssystem, wie einen Staubsauger.

Die beispielhaft gezeigte Basiseinheit 32 weist eine Strömungsmaschine 7 zur Erzeugung des Gasstroms 6 auf, wobei der Gasstrom 6 eingangsseitig durch einen Aktivkohlefilter 9 und anschließend in einen Abluftkanal 33 geleitet wird. Die Vorrichtung 1 kann schließlich beispielsweise als Erweiterung in den Abluftkanal 33 eingebracht werden. Dabei wird der bestehende Gasstrom 6 ausgenutzt und durch den Gaseinlass 3 der Vorrichtung 1 eingeleitet, um nach Durchströmen des Desinfektionsraums 5 bzw. der Aerosolerzeugungsanordnung 11 gereinigt wieder aus dem Gasauslass 4 auszutreten. Schließlich kann der durch die Vorrichtung 1 gereinigte Gasstrom 6 durch ein Lüftungsgitter 34 der Basiseinheit 32 an die Umgebung abgegeben werden. Der Abluftkanal 33 kann beispielsweise zumindest abschnittsweise durch eine Gebäudewand 35 verlaufen.

## Patentansprüche

1. Vorrichtung (1) zur Reduzierung einer Schadstoffbelastung in einem Gas (2), aufweisend einen Gaseinlass (3) und einen Gasauslass (4), sowie einen dazwischen angeordneten Desinfektionsraum (5), der von einem von dem Gaseinlass (3) zu dem Gasauslass (4) verlaufenden Gasstrom (6) durchströmbar ist, wobei eine ein kapillares Material (10) aufweisende Aerosolerzeugungsanordnung (11) innerhalb des Desinfektionsraums (5) von dem Gasstrom (6) durchströmbar angeordnet ist, wobei sich das kapillare Material (10) in ein Flüssigkeitsreservoir (14) erstreckt, in dem ein flüssiges Oxidationsmittel (15) enthalten ist, und wobei das kapillare Material (10) innerhalb der Aerosolerzeugungsanordnung (11) derart angeordnet ist, dass der Gasstrom (6) zumindest im Wesentlichen parallel an einer mit dem flüssigen Oxidationsmittel (15) benetzten Oberfläche des kapillaren Materials (10) vorbeizuströmen vermag, wobei wenigstens eine Strahlungsquelle (18) zur Erzeugung einer ionisierenden Strahlung innerhalb des Desinfektionsraums (5) angeordnet ist,
**dadurch gekennzeichnet, dass**
a) wenigstens eine Innenwandung des Desinfektionsraums (5) zumindest teilweise mit dem kapillaren Material (10) der Aerosolerzeugungsanordnung (11) ausgekleidet ist; und/oder
b) die Aerosolerzeugungsanordnung (11) wenigstens einen aus dem kapillaren Material (10) gebildeten länglichen Körper (13) aufweist, der sich durch den Desinfektionsraum (5) erstreckt.

2. Vorrichtung (1) nach Anspruch 1,
**dadurch gekennzeichnet, dass**
das kapillare Material (10) ein poröses, anorganisches Material ist, vorzugsweise ein textiles Glasfasergebilde.

3. Vorrichtung (1) nach Anspruch 1 oder 2,
**dadurch gekennzeichnet, dass**
die Strahlungsquelle zur Erzeugung der ionisierenden Strahlung eine UV-C-Strahlungsquelle (18) zur Erzeugung einer UV-C-Strahlung ist.

4. Vorrichtung (1) nach einem der Ansprüche 1 bis 3,
**dadurch gekennzeichnet, dass**
das flüssige Oxidationsmittel (15) Wasserstoffperoxid ist oder Wasserstoffperoxid aufweist.

5. Vorrichtung (1) nach einem der Ansprüche 1 bis 4,
**dadurch gekennzeichnet, dass**
das Flüssigkeitsreservoir (14) an einem unteren Ende des Desinfektionsraums (5) ausgebildet wird.

6. Vorrichtung (1) nach einem der Ansprüche 1 bis 5,
**gekennzeichnet durch**
einen Flüssigkeitstank (16) für das flüssige Oxidationsmittel (15), der mit dem Flüssigkeitsreservoir (14) fluidisch verbunden ist, um dem Flüssigkeitsreservoir (14) bedarfsweise weiteres Oxidationsmittel (15) zuzuführen.

7. Vorrichtung (1) nach einem der Ansprüche 1 bis 6,
**gekennzeichnet durch**
einen in dem Gasstrom (6) angeordneten mechanischen Filter und/oder Aktivkohlefilter (9).

8. Vorrichtung (1) nach einem der Ansprüche 1 bis 7,
**gekennzeichnet durch**
wenigstens eine Strömungsmaschine (7) zur Erzeugung des Gasstroms (6) zwischen dem Gaseinlass (3) und dem Gasauslass (4).

9. Vorrichtung (1) nach einem der Ansprüche 1 bis 8,
**gekennzeichnet durch**
wenigstens ein zwischen dem Gaseinlass (3) und dem Gasauslass (4) angeordnetes Schallabsorberelement (28), vorzugsweise innerhalb des Desinfektionsraums (5), das
a) in dem Gasstrom (6) positioniert ist, so dass der Gasstrom (6) gegen das Schallabsorberelement (28) strömt und von dem Schallabsorberelement (28) abgelenkt wird; und/oder
b) angrenzend an eine Innenwandung der Vorrichtung (1) verläuft, so dass der Gasstrom (6) lateral an dem Schallabsorberelement (28) entlangzuströmen vermag.

10. Vorrichtung (1) nach Anspruch 9,
**dadurch gekennzeichnet, dass**
das wenigstens eine Schallabsorberelement (28) zumindest abschnittsweise in einer Rückhalteeinrichtung (29) aufgenommen ist, wobei die Rückhalteeinrichtung (29) eine dem Gasstrom (6) zugewandte Gitterfläche (30) ausbildet, hinter der das Schallabsorberelement (28) angeordnet ist.

11. Vorrichtung (1) nach Anspruch 9 oder 10,
**dadurch gekennzeichnet, dass**
zumindest ein Teil des kapillaren Materials (10) der Aerosolerzeugungsanordnung (11) durch wenigstens eines der Schallabsorberelemente (28) gebildet ist.

12. System (31) zur Beeinflussung eines Gasstroms (6), aufweisend eine Basiseinheit (32) und eine Vorrichtung (1) nach einem der Ansprüche 1 bis 11, wobei die Vorrichtung (1) als Erweiterung der Basiseinheit (32) derart fluidisch mit der Basiseinheit (32) verbunden ist, dass ein Gasstrom (6) der Basiseinheit (32) durch den Gaseinlass (3) der Vorrichtung (1) geleitet wird, um in den Desinfektionsraum (5) zu gelangen und um aus dem Gasauslass (4) schließlich wieder auszutreten.

13. Verfahren zur Reduzierung einer Schadstoffbelastung in einem Gas (2), wobei ein Desinfektionsraum (5) von einem Gasstrom (6) durchströmt wird, wobei eine ein kapillares Material (10) aufweisende Aerosolerzeugungsanordnung (11) innerhalb des Desinfektionsraums (5) von dem Gasstrom (6) durchströmt wird, wobei sich das kapillare Material (10) in ein Flüssigkeitsreservoir (14) erstreckt, in dem ein flüssiges Oxidationsmittel (15) enthalten ist, und wobei das kapillare Material (10) innerhalb der Aerosolerzeugungsanordnung (11) derart angeordnet ist, dass der Gasstrom (6) zumindest im Wesentlichen parallel an einer mit dem flüssigen Oxidationsmittel (15) benetzten Oberfläche des kapillaren Materials (10) vorbeizuströmen vermag, wobei wenigstens eine Strahlungsquelle (18) zur Erzeugung einer ionisierenden Strahlung innerhalb des Desinfektionsraums (5) verwendet wird,
**dadurch gekennzeichnet, dass**
a) wenigstens eine Innenwandung des Desinfektionsraums (5) zumindest teilweise mit dem kapillaren Material (10) der Aerosolerzeugungsanordnung (11) ausgekleidet ist; und/oder
b) die Aerosolerzeugungsanordnung (11) wenigstens einen aus dem kapillaren Material (10) gebildeten länglichen Körper (13) aufweist, der sich durch den Desinfektionsraum (5) erstreckt.

## Claims

1. Device (1) for reducing a pollutant load in a gas (2), comprising a gas inlet (3) and a gas outlet (4), and a disinfection chamber (5) arranged therebetween, through which a gas flow (6) extending from the gas inlet (3) to the gas outlet (4) can flow, wherein an aerosol generating arrangement (11) comprising a capillary material (10) is arranged inside the disinfection chamber (5) so that the gas flow (6) can flow through it, wherein the capillary material (10) extends into a liquid reservoir (14), which contains a liquid oxidant (15), and wherein the capillary material (15) is arranged inside the aerosol generating arrangement (11) such that the gas flow (6) is capable of flowing at least essentially parallel past a surface of the capillary material (10) wetted with the liquid oxidant (15), wherein at least one radiation source (18) for generating ionizing radiation is arranged inside the disinfection chamber (5),
**characterized in that**
a) at least one inner wall of the disinfection chamber (5) is at least partially lined using the capillary material (10) of the aerosol generating arrangement (11); and/or
b) the aerosol generating arrangement (11) comprises at least one elongated body (13), formed from the capillary material (10), which extends through the disinfection chamber (5).

2. Device (1) according to Claim 1,
**characterized in that**
the capillary material (10) is a porous, inorganic material, preferably a textile glass fiber fabric.

3. Device (1) according to Claim 1 or 2, **characterized in that**
the radiation source for generating the ionizing radiation is a UV-C radiation source (18) for generating UV-C radiation.

4. Device (1) according to any one of Claims 1 to 3,
**characterized in that**
the liquid oxidant (15) is hydrogen peroxide or comprises hydrogen peroxide.

5. Device (1) according to any one of Claims 1 to 4,
**characterized in that**
the liquid reservoir (14) is formed at a lower end of the disinfection chamber (5).

6. Device (1) according to any one of Claims 1 to 5,
**characterized by**
a liquid tank (16) for the liquid oxidant (15), which is fluidically connected to the liquid reservoir (14) in order to supply further oxidant (15) to the liquid reservoir (14) as needed.

7. Device (1) according to any one of Claims 1 to 6,
**characterized by**
a mechanical filter and/or activated carbon filter (9) arranged in the gas flow (6).

8. Device (1) according to any one of Claims 1 to 7,
**characterized by**
at least one turbomachine (7) for generating the gas flow (6) between the gas inlet (3) and the gas outlet (4).

9. Device (1) according to any one of Claims 1 to 8,
**characterized by** at least one sound absorber element (28) arranged between the gas inlet (3) and the gas outlet (4), preferably inside the disinfection chamber (5), which
a) is positioned in the gas flow (6) so that the gas flow (6) flows against the sound absorber element (28) and is deflected by the sound absorber element (28); and/or
b) extends adjoining an inner wall of the device (1), so that the gas flow (6) is capable of flowing laterally past the sound absorber element (28).

10. Device (1) according to Claim 9,
**characterized in that**
the at least one sound absorber element (28) is accommodated at least in some sections in a restraint device (29), wherein the restraint device (29) forms a grating surface (30) facing toward the gas flow (6), behind which the sound absorber element (28) is arranged.

11. Device according to Claim 9 or 10,
**characterized in that**
at least a part of the capillary material (10) of the aerosol generating arrangement (11) is formed by at least one of the sound absorber elements (28).

12. System (31) for influencing a gas flow (6), comprising a base unit (32) and a device (1) according to any one of Claims 1 to 11, wherein the device (1) is fluidically connected to the base unit (32) as an expansion of the base unit (32) such that a gas flow (6) of the base unit (32) is conducted through the gas inlet (3) of the device (1) in order to reach the disinfection chamber (5) and finally to emerge again from the gas outlet (4).

13. Method for reducing a pollutant load in a gas (2), wherein a gas flow (6) flows through a disinfection chamber (5), wherein the gas flow (6) flows through an aerosol generating arrangement (11) comprising a capillary material (10) inside the disinfection chamber (5), wherein the capillary material (10) extends into a liquid reservoir (14), which contains a liquid oxidant (15) and wherein the capillary material (15) is arranged inside the aerosol generating arrangement (11) such that the gas flow (6) is capable of flowing at least essentially parallel past a surface of the capillary material (10) wetted with the liquid oxidant (15), wherein at least one radiation source (18) for generating ionizing radiation is arranged inside the disinfection chamber (5),
**characterized in that**
a) at least one inner wall of the disinfection chamber (5) is at least partially lined using the capillary material (10) of the aerosol generating arrangement (11); and/or
b) the aerosol generating arrangement (11) comprises at least one elongated body (13), formed from the capillary material (10), which extends through the disinfection chamber (5).

## Revendications

1. Dispositif (1) de réduction d'une charge en substances nocives dans un gaz (2) comportant une entrée de gaz (3) et une sortie de gaz (4), ainsi qu'un espace de désinfection (5) disposé entre les deux et pouvant être parcouru par un flux de gaz (6) s'écoulant de l'entrée de gaz (3) vers la sortie de gaz (4), dans lequel un ensemble de génération d'aérosol (11) comportant un matériau capillaire (10) est disposé à l'intérieur de l'espace de désinfection (5) de manière à pouvoir être traversé par le flux de gaz (6), dans lequel le matériau capillaire (10) s'étend dans un bac à liquide (14) contenant un agent oxydant liquide (15), et dans lequel le matériau capillaire (10) est disposé à l'intérieur de l'ensemble de génération d'aérosol (11), de sorte que le flux de gaz (6) peut s'écouler au moins sensiblement parallèlement sur une surface du matériau capillaire (10) mouillée avec l'agent oxydant liquide (15), adans lequel au moins une source de rayonnement (18) pour la génération d'un rayonnement ionisant est disposée à l'intérieur de l'espace de désinfection (5),
**caractérisé en ce que**,
a) au moins une paroi intérieure de l'espace de désinfection (5) est revêtue au moins en partie du matériau capillaire (10) de l'ensemble de génération d'aérosol (11) ;
et/ou
b) l'ensemble de génération d'aérosol (11) comporte au moins un corps allongé (13) formé dans le matériau capillaire (10), qui s'étend à travers l'espace de désinfection (5).

2. Dispositif (1) selon la revendication 1,
**caractérisé en ce que**,
le matériau capillaire (10) est un matériau poreux, inorganique, de préférence une structure textile en fibre de verre.

3. Dispositif (1) selon la revendication 1 ou 2,
**caractérisé en ce que**,
la source de rayonnement pour la génération du rayonnement ionisant est une source de rayonnement UV-C (18) pour la génération d'un rayonnement UV-C.

4. Dispositif (1) selon l'une des revendications 1 à 3,
**caractérisé en ce que**,
l'agent oxydant liquide (15) est du peroxyde d'hydrogène ou comporte du peroxyde d'hydrogène.

5. Dispositif (1) selon l'une des revendications 1 à 4,
**caractérisé en ce que**,
le bac à liquide (14) est aménagé à une extrémité inférieure de l'espace de désinfection (5).

6. Dispositif (1) selon l'une des revendications 1 à 5,
**caractérisé par**
un réservoir de liquide (16) pour l'agent oxydant liquide (15), qui est relié de manière fluidique avec le bac à liquide (14), afin d'alimenter en cas de besoin le bac à liquide (14) en agent oxydant (15) supplémentaire.

7. Dispositif (1) selon l'une des revendications 1 à 6,
**caractérisé par**
un filtre mécanique et//ou un filtre à charbon actif (9) disposé dans le flux de gaz (6).

8. Dispositif (1) selon l'une des revendications 1 à 7,
**caractérisé par**
au moins une turbomachine (7) pour la génération du flux de gaz (6) entre l'entrée de gaz (3) et la sortie de gaz (4).

9. Dispositif (1) selon l'une des revendications 1 à 8,
**caractérisé par**
au moins un élément d'absorption du bruit (28) disposé entre l'entrée de gaz (3) et la sortie de gaz (4), de préférence à l'intérieur de l'espace de désinfection (5), qui
a) est positionné dans le flux de gaz (6) de sorte que le flux de gaz (6) s'écoule contre l'élément d'absorption du bruit (28) et est dévié par l'élément d'absorption du bruit (28) ;
et/ou
b) s'étend de manière adjacente à une paroi intérieure du dispositif (1), de sorte que le flux de gaz (6) peut s'écouler latéralement le long de l'élément d'absorption du bruit (28).

10. Dispositif (1) selon la revendication 9,
**caractérisé en ce que**,
ledit au moins un élément d'absorption du bruit (28) est logé au moins en partie dans un dispositif de retenue (29), dans lequel le dispositif de retenue (29) forme une surface grillagée (30) orientée vers le flux de gaz (6), derrière laquelle est disposé l'élément d'absorption du bruit (28).

11. Dispositif (1) selon la revendication 9 ou 10,
**caractérisé en ce que**,
au moins une partie du matériau capillaire (10) de l'ensemble de génération d'aérosol (11) est formé au moins par l'un des éléments d'absorption du bruit (28).

12. Système (31) pour influencer un flux de gaz (6), comportant une unité de base (32) et un dispositif (1) selon l'une des revendications 1 à 11, dans lequel le dispositif (1) en extension de l'unité de base (32), est en liaison fluidique avec l'unité de base (32), de sorte qu'un flux de gaz (6) de l'unité de base (32) est dirigé à travers l'entrée de gaz (3) du dispositif (1), pour pénétrer dans l'espace de désinfection (5) et finalement quitter ce dernier par la sortie de gaz (4).

13. Procédé de réduction d'une charge en substances nocives dans un gaz (2), dans lequel un flux de gaz (6) s'écoule à travers un espace de désinfection (5), dans lequel un ensemble de génération d'aérosol (11) comportant un matériau capillaire (10) est traversé par le flux de gaz (6) à l'intérieur de l'espace de désinfection (5), dans lequel le matériau capillaire (10) s'étend dans un bac à liquide (14) contenant un agent oxydant liquide (15), et dans lequel le matériau capillaire (10) est disposé à l'intérieur de l'ensemble de génération d'aérosol (11), de sorte que le flux de gaz (6) peut s'écouler au moins sensiblement parallèlement sur une surface du matériau capillaire (10) mouillée avec l'agent oxydant liquide (15), dans lequel au moins une source de rayonnement (18) pour la génération d'un rayonnement ionisant à l'intérieur de l'espace de désinfection (5) est utilisée,
**caractérisé en ce que**,
a) au moins une paroi intérieure de l'espace de désinfection (5) est revêtue au moins en partie du matériau capillaire (10) de l'ensemble de génération d'aérosol (11) ;
et/ou
b) l'ensemble de génération d'aérosol (11) comporte au moins un corps allongé (13) formé dans le matériau capillaire (10), qui s'étend à travers l'espace de désinfection (5).
